# EUROPEAN PATENT APPLICATION

(11) **EP 4 837 613 A1**
(43) Date of publication of application: **11.11.2026**
(21) Application number: 25752249.0
(22) Date of filing: 07.02.2025
(51) Int. Cl.: A61M 25/06, A61M 5/145

(54) **NASAL ADMINISTRATION DEVICE AND NASAL ADMINISTRATION SYSTEM**

(30) Priority: 08.02.2024 JP 2024017856
(71) Applicant: Terumo Kabushiki Kaisha, Tokyo 151-0072 (JP)
(72) Inventor: SATO, Eri, Ashigarakami-gun, Kanagawa 259-0151 (JP); ARINOBE, Manabu, Ashigarakami-gun, Kanagawa 259-0151 (JP); NAKAOKA, Mayu, Ashigarakami-gun, Kanagawa 259-0151 (JP); HATA, Suguru, Ashigarakami-gun, Kanagawa 259-0151 (JP); IWASE, Yoichiro, Ashigarakami-gun, Kanagawa 259-0151 (JP)
(74) Representative: Prüfer & Partner mbB Patentanwälte · Rechtsanwälte
(86) International application number: PCT/JP2025/004073
(87) International publication number: WO 2025/170026

(57) **Abstract**

An object of the present invention is to effectively deliver an administered substance into the brain of a mammal in a minimally invasive manner.

There is provided an intranasal administration device 1 including a needle portion 10 having a puncture portion 12 for delivering an administered substance A intranasally into the brain of a mammal, in which the administered substance A is administered into the brain through an opening portion of the puncture portion 12 in a state where the opening portion (distal end opening portion 14) is disposed within a cribriform foramen X3 and a ratio of a puncture length of a distal end of the opening portion (distal end opening portion 14) of the puncture portion 12 into the cribriform foramen X3 to a total length between a nasal-cavity-side opening and an olfactory-bulb-side opening of the cribriform foramen X3 is 60% or more and 100% or less.

## Description

### Technical Field

The present invention relates to an intranasal administration device and an intranasal administration system for intranasally administering an administered substance such as a drug and delivering the administered substance into the brain of a mammal.

### Background Art

In the brain, transport of substances between circulating blood and brain tissue is strictly controlled by the blood-brain barrier (BBB), and particularly when macromolecular drugs (such as proteins and antibodies) are administered orally or intravenously, the amount of drug delivered to the brain is extremely limited.

As methods for administering a drug into the brain more effectively than oral administration or intravenous administration, research has been conducted on drug transfer via a cerebrospinal fluid (CSF), and at present, methods such as an intranasal spray method in which a drug is sprayed into the nasal cavity, an intraventricular administration method in which a drug is directly administered into a cerebral ventricle, and an intrathecal administration method in which a drug is injected into the spinal cavity are known.

However, although the intranasal spray method is minimally invasive, the drug transfer rate is extremely low. In the intraventricular administration method, because the skull is perforated and the drug is directly administered into a cerebral ventricle, there is a risk of injury caused by cerebral puncture. Although the intrathecal administration method provides a higher drug transfer rate than the intranasal spray method, the rate is not sufficient to obtain a pharmacological effect, and because the method cannot be regarded as minimally invasive, many issues remain for practical application.

Patent Literature 1 also discloses a drug delivery device that gradually releases a drug under the olfactory mucosa based on the problem caused by the restriction of drug transfer through the BBB.

### Citation List

### Patent Literature

Patent Literature 1: US 2021/0023295 A

### Summary of Invention

### Technical Problem

The method of Patent Literature 1 provides sustained drug release under the olfactory mucosa. However, the olfactory mucosa is composed of the olfactory epithelium and the lamina propria, and a large number of blood vessels and lymphatic vessels are present in the lamina propria. Therefore, the drug is easily absorbed into blood vessels and lymphatic vessels in the lamina propria, and in particular, the lymphatic vessels cause the drug to flow in a direction away from the brain, and thus the amount transferred into the brain is limited.

The inventors of the present application and others considered that there was room for improvement in intranasal administration, for which the above-described intranasal spray and sub-olfactory mucosal administration are known as minimally invasive intracerebral drug delivery routes, and studied methods capable of improving the drug transfer rate into the brain. During repeated studies, the inventors of the present application and others hypothesized that the low delivery efficiency into the brain was caused by the flow of CSF seeping from the brain into the nasal cavity, which inhibits delivery of the drug to the brain, and proceeded with research on a novel intranasal administration method capable of avoiding this inhibitory factor. As a result, the inventors of the present application and others discovered that delivery efficiency of an administered substance into the brain is dramatically improved by directly administering a drug into a cribriform foramen formed in the cribriform plate and, in addition, by appropriately controlling the puncture ratio of a distal end opening portion formed in the needle portion of the device relative to the cribriform foramen, thereby leading to the development of the invention of the present application.

At least one embodiment of the present invention has been made in view of the above circumstances, and specifically, an object thereof is to provide an intranasal administration device and an intranasal administration system capable of effectively delivering an administered substance into the brain of a mammal in a minimally invasive manner.

### Solution to Problem

The above object of the present invention is achieved by any one of the following (1) to (9).
(1) An intranasal administration device including a needle portion having a puncture portion for delivering an administered substance intranasally into the brain of a mammal, in which the administered substance is administered into the brain through an opening portion of the puncture portion in a state where the opening portion is disposed within a cribriform foramen and a ratio of a puncture length of a distal end of the opening portion into the cribriform foramen to a total length between a nasal-cavity-side opening and an olfactory-bulb-side opening of the cribriform foramen is 60% or more and 100% or less.
(2) The intranasal administration device according to the above (1), in which the intranasal administration device has a cannula portion that is constituted by a tubular member and is disposed to cover the needle portion such that the puncture portion is exposed, the opening portion is provided on a distal end side of the puncture portion, a stopper portion having an abutment portion that abuts against an olfactory epithelium of an olfactory mucosa is formed at a distal end portion of the cannula portion, and the opening portion is disposed within the cribriform plate in a state where the abutment portion of the stopper portion is in contact with the olfactory epithelium.
(3) The intranasal administration device according to the above (2), in which the intranasal administration device has a hub portion to which an accommodation portion containing the administered substance is attachable, and the hub portion holds a proximal end of the cannula portion and/or a proximal end of a needle shaft portion of the needle portion inserted through a lumen of the cannula portion.
(4) The intranasal administration device according to any one of the above (1) to (3), in which a needle tip portion of the puncture portion is an open end provided with a blade surface on a distal end side thereof, and a total length of the puncture portion is 0.35 mm or more and 5.4 mm or less.
(5) The intranasal administration device according to the above (2), in which a total length of the cannula portion is 55 mm or more and 410 mm or less.
(6) The intranasal administration device according to the above (2) or (3), in which the abutment portion of the stopper portion is a distal end surface of the cannula portion that comes into contact with the olfactory epithelium, a length of a longest portion in a radial direction of the abutment portion is 0.2 mm or more and 15 mm or less, and an outer diameter on a proximal end side of the puncture portion is 0.1 mm or more and 2.1 mm or less.
(7) The intranasal administration device according to the above (2) or (6), in which a cross-sectional shape of the abutment portion of the stopper portion is circular or elliptical.
(8) The intranasal administration device according to the above (1) or (2), in which the intranasal administration device has an accommodation portion accommodating the administered substance and is configured such that the administered substance accommodated in the accommodation portion is dischargeable through the opening portion of the puncture portion.
(9) An intranasal administration system including: the intranasal administration device according to any one of the above (1) to (8); and a guide catheter having a lumen into and from which at least a part of the intranasal administration device is insertable and removable, the guide catheter guiding the needle portion of the intranasal administration device inserted into the lumen to the cribriform foramen formed in the cribriform plate.

### Advantageous Effects of Invention

According to the present invention, the administered substance can be administered into the brain through the distal end opening portion in a state where the distal end opening portion of the puncture portion is disposed in the cribriform foramen and the puncture ratio of the distal end of the distal end opening portion relative to the total length of the cribriform foramen is controlled to 60% or more and 100% or less. Therefore, the administered substance can be efficiently delivered to brain tissue in a minimally invasive manner via a delivery medium such as a cerebrospinal fluid or an olfactory nerve. In particular, the present invention is extremely effective because a therapeutic macromolecular drug such as a protein or antibody, which is currently difficult to administer to brain tissue, can be efficiently delivered to the brain tissue while bypassing the blood-brain barrier or the like. In addition, according to the present invention, since the administered substance is administered within a cribriform foramen formed in a cribriform plate after passing through a lamina propria of an olfactory mucosa in which a large number of blood vessels and lymphatic vessels are present, the administered substance can be delivered to brain tissue while suppressing leakage into a nasal cavity and minimizing an amount flowing into the blood vessels and the lymphatic vessels.

### Brief Description of Drawings

[Fig. 1] Fig. 1 is a view illustrating an intranasal administration system according to the present embodiment.
[Fig. 2] Fig. 2 is a view illustrating a state where a needle tip portion of the intranasal administration device punctures a cribriform foramen.
[Fig. 3A] Fig. 3A is a view illustrating an example of use of the intranasal administration device according to the present embodiment.
[Fig. 3B] Fig. 3B is a view illustrating an example of use of the intranasal administration device according to the present embodiment.
[Fig. 3C] Fig. 3C is a view illustrating an example of use of the intranasal administration device according to the present embodiment.
[Fig. 3D] Fig. 3D is a view illustrating an example of use of the intranasal administration device according to the present embodiment.
[Fig. 3E] Fig. 3E is a view illustrating an example of use of the intranasal administration device according to the present embodiment.
[Fig. 4] Fig. 4 is a view illustrating an intranasal administration device produced in Test 1.
[Fig. 5] Fig. 5 is a table showing results of Test 1.
[Fig. 6] Fig. 6 is a graph illustrating results of Test 1.
[Fig. 7] Fig. 7 is a view for explaining a contrast position in a CT image of Test 1.
[Fig. 8] Fig. 8 is a CT image after administration of an administered substance (contrast agent) to Sample 14, which is an Example of Test 1.
[Fig. 9] Fig. 9 is a CT image after administration of an administered substance (contrast agent) to Sample 5, which is a Comparative Example of Test 1.
[Fig. 10] Fig. 10 is a view illustrating an intranasal administration device produced in Test 2.
[Fig. 11] Fig. 11 is a table showing results of Test 2.
[Fig. 12] Fig. 12 is a graph illustrating results of Test 2.
[Fig. 13] Fig. 13 is a view for explaining a contrast position in a CT image of Test 2.
[Fig. 14] Fig. 14 is a CT image after administration of an administered substance (contrast agent) to Sample 12, which is an Example of Test 2.
[Fig. 15] Fig. 15 is a CT image after administration of an administered substance (contrast agent) to Sample 3, which is a Comparative Example of Test 2.
[Fig. 16] Fig. 16 is a table showing specifications of intranasal administration devices used in Test 3, Test 4, and Test 5, and test results of each test.
[Fig. 17] Fig. 17 is a CT image after administration of an administered substance (contrast agent) to Sample 14, which is an Example of Test 3.
[Fig. 18] Fig. 18 is a CT image after administration of an administered substance (contrast agent) to Sample 15, which is an Example of Test 4.
[Fig. 19] Fig. 19 is a view illustrating an intranasal administration device used in Test 5.
[Fig. 20] Fig. 20 is a CT image after administration of an administered substance (contrast agent) to Sample 16, which is an Example of Test 5.

### Description of Embodiments

Hereinafter, aspects for carrying out the present invention will be described in detail with reference to the drawings. Embodiments described herein are illustrated to embody the technical idea of the present invention and do not limit the present invention. Furthermore, other practicable aspects, examples, technical operations and the like that can be conceived by those skilled in the art without departing from the gist of the present invention are all included within the scope and gist of the present invention and included within the scope of the invention described in claims and the equivalent scope thereof.

Furthermore, for convenience of illustration and ease of understanding, the drawings attached to the present specification may be schematically expressed by appropriately converting scales, vertical and horizontal dimensional ratios, shapes, and the like from actual objects, but are merely examples, and do not limit the interpretation of the present invention.

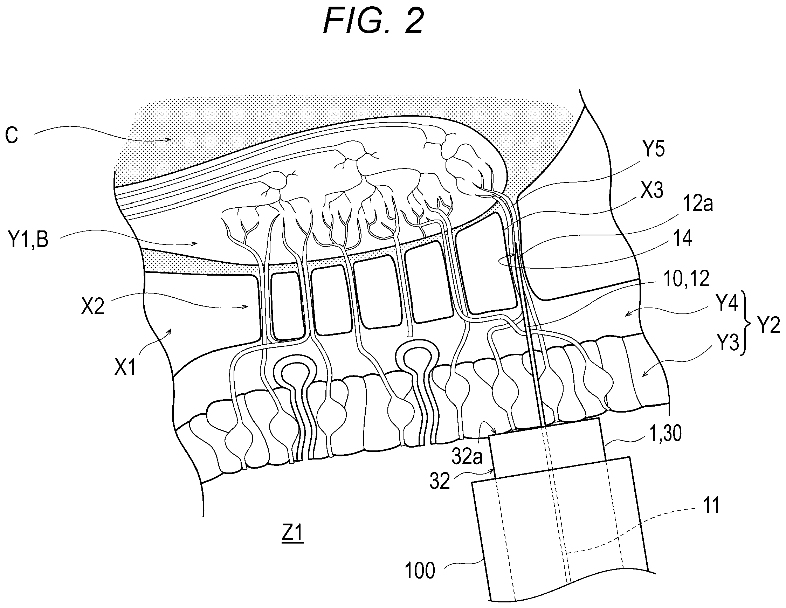

In the present specification, the "cribriform foramen X3" punctured by a puncture portion 12 of a needle portion 10 of an intranasal administration device 1 is a hole formed in a cribriform plate X2 of an ethmoid bone X1 and having a nasal-cavity-side opening portion and an olfactory-bulb-side opening portion as illustrated in Fig. 2, and an olfactory nerve (nerve axon) Y5 extending from an olfactory bulb Y1, which is a part of a brain tissue B, to olfactory cells distributed in an olfactory mucosa Y2 (composed of an olfactory epithelium Y3 and a lamina propria Y4) in a nasal cavity Z1 passes therethrough. A large number of cribriform foramina X3 are present not only on the flat surface of the cribriform plate X2 as illustrated in Fig. 2, but also in the midline, the lateral wall, the posterior wall of the olfactory fossa, and the like. The cribriform foramen X3, which is a puncture target of the needle portion 10 of the present device, is any hole, among all of these holes, that can be punctured by the puncture portion 12 having penetrated through the olfactory mucosa Y2.

### [Intranasal Administration System]

An intranasal administration system 200 according to the present embodiment will be described.

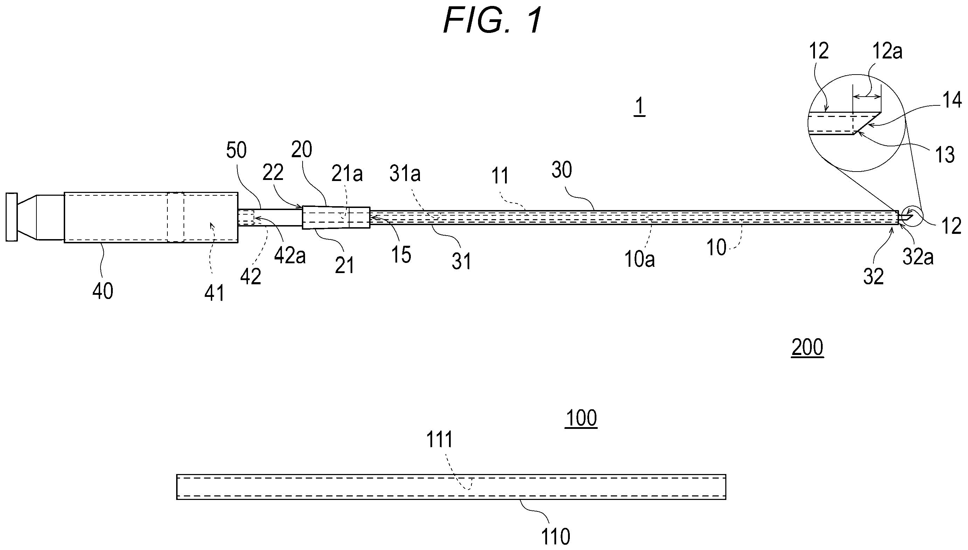

As illustrated in Fig. 1, the intranasal administration system 200 is configured to include the intranasal administration device 1 and a guide catheter 100.

The intranasal administration system 200 administers an administered substance A into the brain in a state where the distal end side of the guide catheter 100 is disposed within the nasal cavity Z1 in a posture of being oriented toward the cribriform plate X2, the intranasal administration device 1 is inserted into the guide catheter 100, the opening portion (distal end opening portion 14) of the puncture portion 12 of the needle portion 10 is disposed within the cribriform foramen X3 formed in the cribriform plate X2, and a ratio of a puncture length of a distal end of the distal end opening portion 14 into the cribriform foramen X3 to the total length of the cribriform foramen X3 (a length of a shortest portion between a nasal-cavity-side opening and an olfactory-bulb-side opening of the cribriform foramen X3) is 60% or more and 100% or less. The intranasal administration system 200 can deliver the administered substance A intranasally into the brain of a mammal such as a human via a delivery medium such as a cerebrospinal fluid C or the olfactory nerve Y5.

### <Intranasal Administration Device>

As illustrated in Fig. 1 or 2, the intranasal administration device 1 is configured to include the needle portion 10, a hub portion 20, a cannula portion 30, and an accommodation portion 40.

The intranasal administration device 1 is an intranasal administration device 1 including the needle portion 10 having the puncture portion 12 for delivering the administered substance A intranasally into the brain of a mammal, in which the administered substance A is administered through the distal end opening portion 14 in a state where the opening portion (distal end opening portion 14) of the puncture portion 12 is disposed within the cribriform foramen X3 and a ratio of a puncture length of a distal end of the distal end opening portion 14 into the cribriform foramen X3 to the total length of the cribriform foramen X3 is 60% or more and 100% or less. The intranasal administration device 1 can also be said to be "an intranasal administration device for delivering an administered substance intranasally into the brain of a mammal, the intranasal administration device being configured such that an administered substance A is administered in a state where a distal end opening portion provided in a puncture portion disposed at a distal end is disposed within a cribriform foramen and a ratio of a puncture length of the distal end of the distal end opening portion into the cribriform foramen to the total length between a nasal-cavity-side opening and an olfactory-bulb-side opening of the cribriform foramen is 60% or more and 100% or less", or "an intranasal administration device for use in an intranasal administration method for delivering an administered substance intranasally into the brain of a mammal using the intranasal administration device, the intranasal administration device including a puncture portion protruding toward a distal end side and a distal end opening portion provided in the puncture portion, in which the intranasal administration method includes administering the administered substance into the brain in a state where the distal end opening portion is disposed within the cribriform foramen and a ratio of a puncture length of the distal end of the distal end opening portion into the cribriform foramen to a total length between a nasal-cavity-side opening and an olfactory-bulb-side opening of the cribriform foramen is 60% or more and 100% or less".

In the intranasal administration device 1 of the present invention, the administered substance A is delivered into the brain by administering the administered substance A into the cribriform foramen X3 through the distal end opening portion 14 in a state where the distal end opening portion 14 of the puncture portion 12 is disposed within the cribriform foramen X3. This administration method is a novel method obtained as a result of intensive studies conducted by the inventors of the present application and others to avoid this inhibitory factor, based on a hypothesis that the cause of an extremely low drug transfer rate to the brain in a known minimally invasive intranasal administration method is that a flow of the cerebrospinal fluid C seeping from the brain into the nasal cavity Z1 inhibits delivery of the drug to the brain. When the administered substance A is administered into the cribriform foramen X3 using the present device, the transfer rate of the administered substance A to the brain is dramatically improved. This mechanism is considered to be due to imparting to the administered substance A a liquid pressure that counteracts the flow of the cerebrospinal fluid C without leakage into the nasal cavity Z1 by administering the administered substance A in a state where the needle portion 10 is punctured into the cribriform foramen X3. Therefore, the administered substance A administered in the cribriform foramen X3 through the distal end opening portion 14 flows toward the brain tissue B side through the olfactory-bulb-side opening of the cribriform foramen X3 against the flow of the cerebrospinal fluid C, and can be delivered to the brain at an extremely high transfer rate via a delivery medium such as the cerebrospinal fluid C or the olfactory nerve Y5.

Furthermore, although the intranasal administration device 1 administers the administered substance A in a state where the distal end opening portion 14 of the puncture portion 12 is disposed within the cribriform foramen X3, the ratio of the puncture length (puncture amount) of the distal end of the distal end opening portion 14 to the total length of the cribriform foramen X3 is also extremely important for delivering the administered substance A to the brain tissue B with high efficiency. This "puncture ratio" is obtained from the ratio of the puncture length of the distal end of the distal end opening portion 14 into the cribriform foramen X3 to the "total length of the cribriform foramen X3", which is the length of the shortest portion between the nasal-cavity-side opening and the olfactory-bulb-side opening in the thickness direction of the cribriform foramen X3 (that is, a ratio of a puncture length of the needle portion 10 including the distal end opening portion 14 from the most distal end of the needle portion 10 toward the proximal end, which is a length by which the needle portion 10 is punctured into the cribriform foramen X3).

When the administered substance A is administered into the cribriform foramen X3, if administration is performed in a state where the distal end opening portion 14 is disposed on the nasal-cavity-side opening side of the cribriform foramen X3, a liquid pressure sufficient to counteract the flow of the cerebrospinal fluid C seeping out from the cribriform foramen X3 of the cerebrospinal fluid C is imparted. However, because the administration position is close to the nasal-cavity-side opening, there is a possibility that a part of the administered substance A leaks toward the nasal cavity Z1 side, and there is a concern that a sufficient effect cannot be obtained.

In the intranasal administration device 1, in order to avoid such problems and efficiently deliver the administered substance A to the brain while preventing leakage into the nasal cavity 21, the distal end opening portion 14 is disposed within the cribriform foramen X3 and the puncture ratio of the distal end of the distal end opening portion 14 into the cribriform foramen X3 relative to the total length of the cribriform foramen X3 is set to 60% or more and 100% or less. In the intranasal administration device 1, when the puncture ratio of the distal end of the distal end opening portion 14 relative to the cribriform foramen X3 is 60% or more, the administered substance A can be reliably administered into the cribriform foramen X3 without leaking into the nasal cavity Z1, and when the puncture ratio is 100% or less, damage to the brain tissue B caused by penetrating through the olfactory-bulb-side opening of the cribriform foramen X3 can be prevented.

In addition, the above-described range of the puncture ratio of the distal end opening portion 14 relative to the cribriform foramen X3 (60% or more and 100% or less) is set in order to achieve 100% successful delivery of the administered substance A to the brain tissue B side with no or extremely little leakage of the administered substance A, which was administered, into the nasal cavity Z1. That is, puncturing the distal end opening portion 14 into the cribriform foramen X3 at a puncture ratio of 60% or more means that the administered substance A, which was administered, can be reliably delivered to the brain tissue B side. However, when the administered substance A is administered in a state where the distal end of the distal end opening portion 14 is punctured into the cribriform foramen X3, even if the puncture ratio is less than 60% (for example, 50% or more and 100% or less), the intranasal administration device 1 can deliver the administered substance A to the brain tissue B side at a transfer rate higher than that of conventional intranasal administration methods, even without achieving a 100% success rate as in the case where the puncture ratio is 60% or more. Therefore, in the intranasal administration device 1, delivery of the administered substance A to the brain tissue B is possible even when the puncture ratio of the distal end of the distal end opening portion 14 is less than 60%. However, when administration to humans is assumed, conditions that achieve a 100% success rate are important, and therefore the puncture ratio is preferably 60% or more and 100% or less.

The intranasal administration device 1 can be used in combination with the guide catheter 100 as the intranasal 0administration system 200, or can be used alone.

### <Needle Portion>

The needle portion 10 has a needle shaft portion 11, a puncture portion 12 formed on the distal end side of the needle shaft portion 11, a distal end opening portion 14 formed in a needle tip portion 12a of the puncture portion 12 (corresponding to the "opening portion" in the claims), and a proximal end opening portion 15 formed at the proximal end of the needle shaft portion 11. The needle portion 10 has a cylindrical shape in which a lumen 10a penetrating in the longitudinal direction is formed from the distal end opening portion 14 formed on the distal end side to the proximal end opening portion 15 formed on the proximal end side. As illustrated in Fig. 1, in the needle portion 10, the needle shaft portion 11 is disposed within a lumen 31a of the cannula portion 30, and at the time of puncture, the puncture portion 12 is disposed to be exposed from the distal end of the cannula portion 30.

The needle shaft portion 11 corresponds to a main body portion of the needle portion 10 and has a lumen 21a communicating with the hub portion 20 or the lumen 31a of the cannula portion 30. The proximal end opening portion 15 is formed at the proximal end of the needle shaft portion 11. The needle shaft portion 11 is communicably connected to the hub portion 20 through the proximal end opening portion 15.

The needle shaft portion 11 is formed to have a length substantially equal to the total length of the cannula portion 30, is inserted through the lumen 31a of the cannula portion 30, and is connected to the hub portion 20 such that the proximal end opening portion 15 communicates with the lumen 21a of the hub portion 20. However, the needle shaft portion 11 may be shorter than the total length of the cannula portion 30, and the proximal end may be disposed in the lumen 31a of the cannula portion 30. When configured in this manner, the administered substance A flows through the lumen 31a of the cannula portion 30 to the proximal end opening portion 15.

The puncture portion 12 is formed on the distal end side of the needle shaft portion 11. The needle tip portion 12a is formed on the distal end side of the puncture portion 12. The needle tip portion 12a has an open end provided with a blade surface 13 formed by cutting the needle shaft portion 11 obliquely with respect to the longitudinal direction on the distal end side. An inner edge of the blade surface 13 defines the distal end opening portion 14 that communicates the lumen of the needle shaft portion 11 with the outside. At the time of administration of the administered substance A, the distal end opening portion 14 is disposed within the cribriform foramen X3 in a state where at least a part of the puncture portion 12 is punctured into the cribriform foramen X3. By being disposed within the cribriform foramen X3, the distal end opening portion 14 can deliver the administered substance A toward the brain against the cerebrospinal fluid C flowing out from the cribriform foramen X3. In addition, the needle tip portion 12a is not limited to a pointed shape having the blade surface 13 at the distal end, and may have a straight tubular shape or a tubular shape having a substantially hemispherical rounded distal end.

From the viewpoints of ease of puncture into the cribriform foramen X3 and liquid feedability of the administered substance A, the puncture portion 12 has an outer diameter of 0.1 mm or more and 2.1 mm or less, and more preferably 0.1 mm or more and 1.2 mm or less. The total length of the puncture portion 12, which is the axial length of the blade surface 13 illustrated in Fig. 1 (corresponding to the exposed length of the needle portion 10 from the distal end of the cannula portion 30), has a length that allows the distal end opening portion 14 to be disposed at least within the cribriform foramen X3, and is 0.35 mm or more and 5.4 mm or less. The total length of the puncture portion 12 is the length in the major-axis direction of the portion exposed from the cannula portion 30 of the needle portion 10, and is the length from the most distal end of the needle tip portion 12a along the major-axis direction of the needle portion 10. The length of the puncture portion 12 can be appropriately set according to the thickness of the olfactory mucosa Y2 of the mammal, which is a puncture target, the total length of the cribriform foramen X3, and the like.

The needle portion 10 can be formed of a metal such as stainless steel (for example, SUS304 or SUS316L) or titanium, a resin material, or the like. However, the constituent material of the needle portion 10 is not particularly limited to the above-described materials, as long as the material can be used in the medical field and is suitable for the needle portion 10.

In addition, in order to improve ease of puncture into the cribriform foramen X3, the needle portion 10 may be formed of a plastically deformable material or a shape-memory material, and may be used in an aspect in which the needle shaft portion 11 is deformed by changing the orientation of the puncture portion 12 when the device is used. At this time, the shape of the needle portion 10 may be deformed into any shape immediately before use, may be deformed in advance, or may be further finely adjusted at the time of use in a state of being deformed in advance.

In addition, the needle portion 10 may use different materials for the puncture portion 12 exposed from the cannula portion 30 and for the needle shaft portion 11. For example, the needle portion 10 may be configured such that the puncture portion 12 is formed of a material having rigidity that does not deform or is difficult to deform in order to puncture the olfactory mucosa or the like, and the needle shaft portion 11 is formed of a material deformable in accordance with the shape or the like of the guide catheter 100.

### <Hub Portion>

The hub portion 20 holds the proximal end of the needle shaft portion 11 of the needle portion 10 and/or the proximal end of the cannula portion 30 such that the administered substance A can flow therethrough. The hub portion 20 has a main body portion 21 and a connection portion 22. The hub portion 20 is connected to the accommodation portion 40 in a state where the distal end opening portion 42a of the accommodation portion 40 and the proximal end opening portion 15 of the needle portion 10 are in communication with each other.

The main body portion 21 has the lumen 21a through which the administered substance A can flow, and causes the distal end opening portion 42a of the connected accommodation portion 40 to communicate with the proximal end opening portion 15 of the needle shaft portion 11 of the needle portion 10 or the lumen 31a of the cannula portion 30.

The connection portion 22 is provided on the proximal end side of the hub portion 20, is connected to the accommodation portion 40, and maintains a communication state between the hub portion 20 and the accommodation portion 40. In the present embodiment, the connection portion 22 is in an aspect of being connected to the accommodation portion 40 via a connection member 50 such as a tube. However, the connection portion 22 may be in an aspect in which both the connection portion 22 and the accommodation portion 40 are directly and detachably fitted to each other in accordance with the shape of the distal end side of the accommodation portion 40 (for example, a luer taper type or a luer lock type). The connection portion 22 is not particularly limited as long as the connection portion 22 is in an aspect in which at least the accommodation portion 40 and the hub portion 20 are connected such that the administered substance A can flow therebetween.

### <Cannula Portion>

The cannula portion 30 is a tubular member formed of a flexible material, and has a tubular main body portion 31 in which the lumen 31a communicating from the distal end to the proximal end is formed. As one example, the cannula portion 30 holds at least a part of the needle shaft portion 11 of the needle portion 10.

A stopper portion 32 having an abutment portion 32a that comes into contact with the olfactory epithelium Y3 of the olfactory mucosa Y2 is provided at the distal end of the main body portion 31 of the cannula portion 30. In the aspect illustrated in Fig. 1, the stopper portion 32 is a distal end part of the main body portion 21, and the distal end surface of the main body portion 31 functions as the abutment portion 32a. In the present embodiment, the stopper portion 32 is the distal end part of the main body portion 31, and the abutment portion 32a is the distal end surface of the main body portion 21. However, the stopper portion 32 and the abutment portion 32a are not limited to these configurations. The stopper portion 32 may be constituted by a detachable or fixed separate member that can be disposed on the distal end side of the cannula portion 30, and the abutment portion 32a may be constituted by a portion of the separate member that comes into contact with the olfactory epithelium Y3.

When the puncture portion 12 of the needle portion 10 punctures the cribriform foramen X3, the stopper portion 32 functions as a stopper that prevents the needle tip portion 12a of the puncture portion 12 from puncturing excessively deeply by bringing the abutment portion 32a into contact with the olfactory epithelium Y3 as illustrated in Fig. 2.

Further, when the needle portion 10 is punctured, the stopper portion 32 can stabilize the puncture posture of the intranasal administration device 1 by bringing the abutment portion 32a into contact with the olfactory epithelium Y3. Accordingly, the needle portion 10 can puncture the cribriform foramen X3, which is a puncture target, without positional displacement. In addition, the stopper portion 32 is preferably in a state of being pressed to slightly sink into the olfactory epithelium Y3 such that the puncture posture of the intranasal administration device 1 is fixed.

In the stopper portion 32, when the abutment portion 32a is the distal end surface of the cannula portion 30, in a case where the cross-sectional shape thereof is substantially circular (such as elliptical) close to the structure of the upper nasal cavity, the length of the longest portion in the radial direction (maximum width) of the distal end surface of the cannula portion 30 has a minor axis of 0.2 mm or more and 3.0 mm or less and a major axis of 0.2 mm or more and 15 mm or less. In the abutment portion 32a, when the cross-sectional shape of the cannula portion 30 is circular, the diameter is preferably 0.2 mm or more and 3.0 mm or less, and more preferably 0.2 mm or more and 2.1 mm or less. The maximum width of the abutment portion 32a can be appropriately set to be at least larger than the diameter of the needle portion 10 such that the stopper function of preventing over-puncture of the needle portion 10 into the cribriform foramen X3 is exhibited.

The cannula portion 30 may be formed in a straight tubular shape, but from the viewpoint of ease of puncture of the needle portion 10 into the cribriform foramen X3, the cannula portion 30 may have a shape in which a part thereof is curved in advance. In addition, from the viewpoints of ease of insertion into the nasal cavity Z1 and ease of puncture of the needle portion 10 into the cribriform foramen X3, the cannula portion 30 may be formed of a material that allows a part or all of the main body portion 31 to be plastically deformed into any shape. Thus, when an insertion aid such as the guide catheter 100 for guiding the intranasal administration device 1 to the olfactory mucosa Y2 is used, the cannula portion 30 can be inserted while being deformed along the lumen shape of the guide catheter 100.

The cannula portion 30 has a length such that the proximal end is exposed from the external naris, the distal end is inserted to the olfactory mucosa Y2 in the vicinity of the cribriform plate X2, and the needle tip portion 12a can be operated, and has a total length of 55 mm or more and 410 mm or less.

The cannula portion 30 may be disposed to cover the total length of the needle shaft portion 11 of the needle portion 10, or may be disposed in a state where the proximal end side of the needle shaft portion 11 is exposed.

The cannula portion 30 may be configured to be disposed integrally with the hub portion 20, or may be configured to be attachable to and detachable from the hub portion 20.

### <Accommodation Portion>

The accommodation portion 40 accommodates the administered substance A to be administered to the cribriform foramen X3. The accommodation portion 40 has an accommodation space 41 that accommodates the administered substance A and a liquid feeding portion 42 that sends the administered substance A in the accommodation space 41 to the hub portion 20. In Fig. 1, the accommodation portion 40 is connected to the hub portion 20 via the connection member 50 such as a tube such that the administered substance A can flow therethrough.

The accommodation portion 40 is communicably connected to the needle portion 10 via the hub portion 20. A distal end opening portion 42a of the liquid feeding portion 42 formed at the distal end of the liquid feeding portion 42 communicates with the proximal end opening portion 15 of the needle portion 10 through the lumen 21a of the hub portion 20. As a result, the administered substance A accommodated in the accommodation portion 40 can flow through the needle shaft portion 11. The accommodation portion 40 is preferably configured such that the administration amount of the administered substance A accommodated in the accommodation space 41 can be adjusted, and for example, a syringe having a plunger can be used. In addition, the accommodation portion 40 is not limited to a syringe, and may be any instrument that at least accommodates the administered substance A and allows the administered substance A to flow to the needle portion 10 through the hub portion 20. Further, the accommodation portion 40 may be connected such that the liquid feeding portion 42 and the hub portion 20 can communicate with each other in a state where the accommodation portion 40 is attached to a medical device having an operation portion capable of controlling the discharge amount, discharge timing, and the like of the administered substance A.

The puncture angle at which the intranasal administration device 1 is inserted into the nasal cavity Z1 and the needle portion 10 is punctured into the cribriform foramen X3 is preferably 0° or more and 48° or less with respect to an opening surface of a nasal-cavity-side opening portion of the cribriform foramen X3. Here, the puncture angle of "0°" means a puncture angle in a state of intersecting the opening surface of the nasal-cavity-side opening of the cribriform foramen X3 in a perpendicular direction.

### <Administered Substance>

The administered substance A is accommodated in the accommodation portion 40 and administered intranasally into the brain of a mammal. The administered substance A is administered intranasally into the brain of a mammal, and is released through the distal end opening portion 14 and delivered to the brain tissue B via the cribriform foramen X3 in a state where the distal end opening portion 14 of the puncture portion 12 is disposed in the cribriform foramen X3 and a ratio of a puncture length of the distal end of the distal end opening portion 14 into the cribriform foramen X3 to the total length of the cribriform foramen **X3,** where the length of the shortest portion between the nasal-cavity-side opening and the olfactory-bulb-side opening of the cribriform foramen X3 is defined as the total length of the cribriform foramen X3, is 60% or more and 100% or less. The administered substance A can also be said to be "an administered substance that is administered intranasally into the brain of a mammal through a distal end opening portion using an intranasal administration device having a puncture portion protruding toward a distal end side and a distal end opening portion provided in the puncture portion, in a state where the distal end opening portion is disposed within a cribriform foramen and a ratio of a puncture length of the distal end of the distal end opening portion into the cribriform foramen to the total length of the cribriform foramen, where the length of the shortest portion between a nasal-cavity-side opening and an olfactory-bulb-side opening of the cribriform foramen is defined as the total length of the cribriform foramen, is 60% or more and 100% or less".

The administered substance A is, as one example, a drug (liquid preparation) intended for treatment of a predetermined disease. The drug includes at least one of low-molecular-weight drugs, peptides, proteins, antibodies, and cells. These contained substances are preferably those suitable for administration to the brain tissue B, and peptides, proteins, and antibodies are particularly preferable. As compounds contained in the drug, cerliponase alfa, chloroprocaine hydrochloride, cytarabine, baclofen, ziconotide, Remodulin, nalmefene hydrochloride, idursulfase beta, and the like can be suitably used. Suitable uses of the drug include therapeutic agents for central nervous system (CNS) diseases such as epilepsy, Huntington's disease, and Parkinson's disease, and diagnostic agents such as contrast agents and radiopharmaceuticals. The administered substance A is not limited to a drug intended for treatment, diagnosis, or the like, and may be a composition that can be administered into the brain to improve or enhance the function of the brain tissue B. In addition, the administered substance A may also be encapsulated in a transport carrier such as a microbubble, liposome, or exosome.

### <Guide Catheter>

The guide catheter 100 is used as an insertion aid when the intranasal administration device 1 is inserted into the nasal cavity Z1.

As illustrated in Fig. 1, the guide catheter 100 has a catheter main body 110 constituted by a tubular member having a lumen 111 that penetrates in the longitudinal direction from the distal end to the proximal end.

The guide catheter 100 can be formed of a material such as a metal or resin usable in the medical field, and SUS304 can be used as one example.

The distal end portion of the catheter main body 110 may be provided with a curved portion that is disposed adjacent thereto and curved to move away from the axis of the catheter main body 110 toward the distal end side. In addition, the guide catheter 100 may also be subjected to processing such as a spiral cut in the circumferential direction and various surface treatments for improving insertability, on the outer surface thereof.

As illustrated in Fig. 1, the catheter main body 110 may be used in a straight state, may be configured to be pre-shaped to be easily inserted into the nasal cavity Z1, or may be configured to be plastically deformable by fine adjustment before or during use.

Here, dimensional examples of the guide catheter 100 are illustrated. The guide catheter 100 may have a total length of 400 mm, an outer diameter of 2.1 mm, an inner diameter of 1.9 mm, and a bending angle of the curved portion 113 of 45 degrees. In addition, the guide catheter 100 may also have a total length of 90 mm, an outer diameter of 0.82 mm, an inner diameter of 0.68 mm, and a bending angle of the curved portion 113 of 45 degrees.

The guide catheter 100 can be supplied to the market as the intranasal administration system 200 in combination with the intranasal administration device 1. In addition, the intranasal administration device 1 or the guide catheter 100 can be made to function as the intranasal administration system 200 by using either of the devices of the intranasal administration device 1 or the guide catheter 100, each of which has already been supplied as a single device.

### [Method of Using Device]

Next, a method of using the above-described intranasal administration device 1 will be described.

The method of using the device described below includes a procedure corresponding to a preparation stage until the intranasal administration device 1 is disposed at a predetermined position and administration of the administered substance A is started, and a procedure for administering the administered substance A after the preparation stage. A method of using the intranasal administration device 1 includes: a step of inserting the intranasal administration device 1 at least from an external naris and puncturing the needle portion 10 into the olfactory mucosa Y2 while puncturing the cribriform foramen X3 of the cribriform plate X2, thereby disposing the distal end opening portion 14 of the puncture portion 12 within the cribriform foramen X3; a step of setting a puncture ratio, which is a ratio of a puncture length of the distal end of the distal end opening portion 14 into the cribriform foramen X3 to the total length of the cribriform foramen X3, where the length of the shortest portion between the nasal-cavity-side opening and the olfactory-bulb-side opening of the cribriform foramen X3 is defined as the total length of the cribriform foramen X3, to 60% or more and 100% or less; and a step of administering the administered substance A through the distal end opening portion 14 disposed in the cribriform foramen X3 at the puncture ratio.

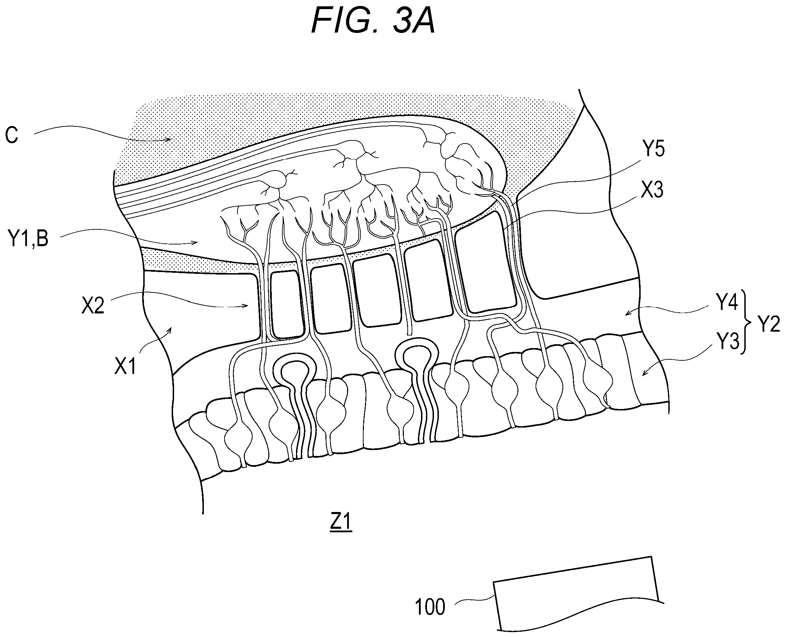

As illustrated in Fig. 3A, a user such as a physician inserts the intranasal administration device 1 from the external naris of a patient toward the olfactory mucosa Y2. When inserting the intranasal administration device 1 into the nasal cavity Z1, the device is inserted while confirming the insertion position using a rigid endoscope or the like in a state where the patient's nose has been anesthetized. The intranasal administration device 1 may be inserted using a straight instrument such as a rigid endoscope, may be directly inserted alone into the nasal cavity Z1, or may be inserted using an insertion aid such as the guide catheter 100.

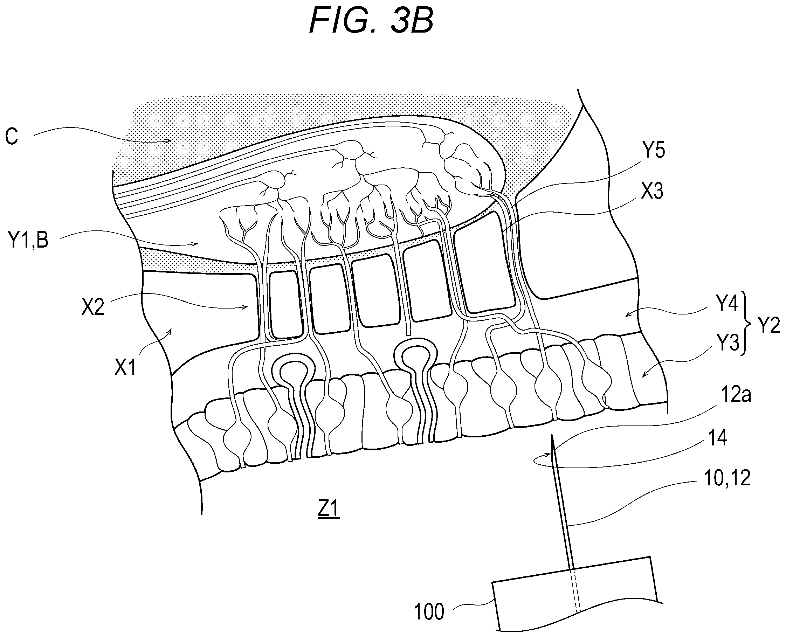

Next, as illustrated in Fig. 3B, the user causes the needle portion 10 to puncture the olfactory mucosa Y2 (in the order of the olfactory epithelium Y3 and the lamina propria Y4).

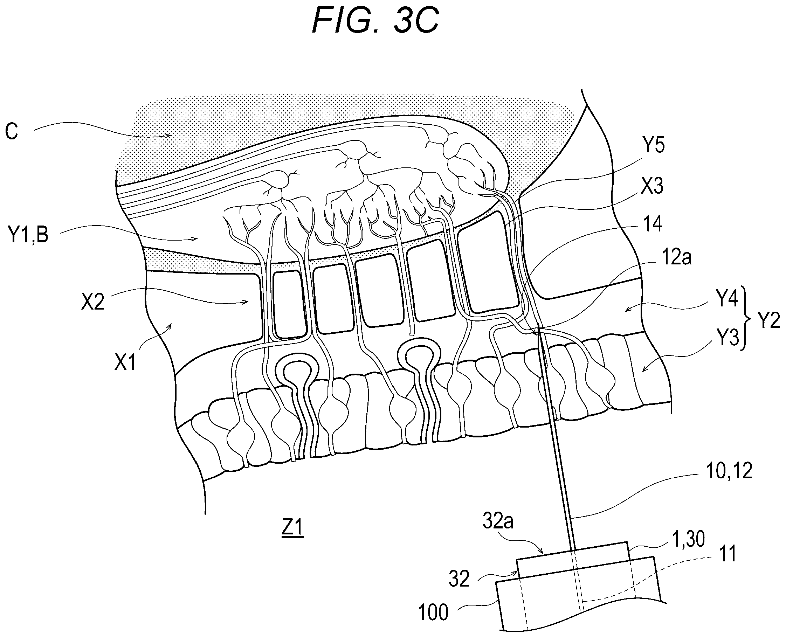

Next, as illustrated in Fig. 3C, the user further advances puncture of the needle portion 10 into the olfactory mucosa Y2 and causes the needle tip portion 12a of the puncture portion 12 to puncture the cribriform foramen X3. At this time, the abutment portion 32a of the stopper portion 32 of the cannula portion 30 comes into contact with the olfactory epithelium Y3, thereby restricting puncturing movement of the needle portion 10 and preventing over-puncture of the needle portion 10 into the cribriform foramen X3. In addition, when the stopper portion 32 is brought into contact with the olfactory epithelium Y3 to be pressed thereagainst, the puncture posture of the intranasal administration device 1 is stabilized, and the puncture portion 12 can be punctured toward the cribriform foramen X3, which is a puncture target, without positional displacement.

In addition, the intranasal administration device 1 is set to a state where the distal end opening portion 14 is disposed within the cribriform foramen X3 and a ratio of a puncture length of the distal end of the distal end opening portion 14 into the cribriform foramen X3 to the total length of the cribriform foramen X3, where the length of the shortest portion between the nasal-cavity-side opening and the olfactory-bulb-side opening of the cribriform foramen X3 is defined as the total length of the cribriform foramen X3, is 60% or more and 100% or less. In the intranasal administration device 1, when a state is achieved where puncturing of the needle portion 10 into the cribriform foramen X3 is completed and the distal end opening portion 14 is disposed within the cribriform foramen X3 at the puncture ratio, administration of the administered substance A can be started.

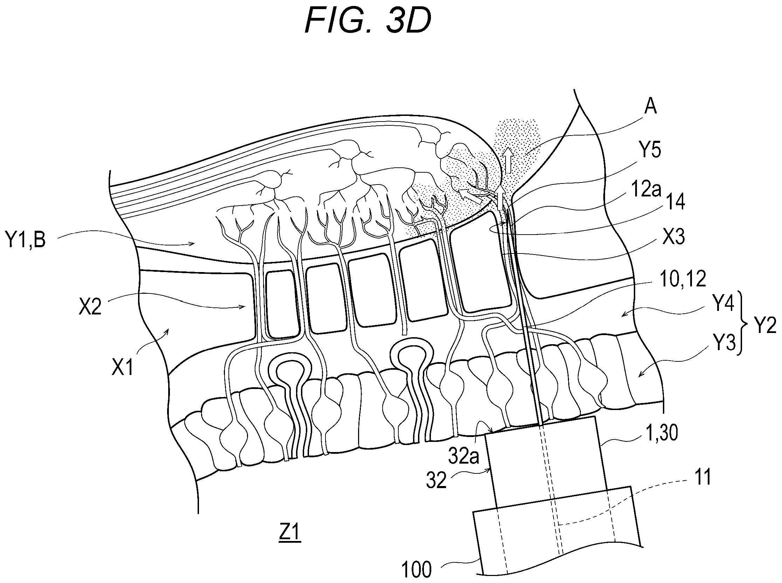

Then, as illustrated in Fig. 3D, the user administers the administered substance A accommodated in the accommodation portion 40. When the accommodation portion 40 is a syringe, the plunger is operated manually or using a syringe pump or the like, and the administered substance A is appropriately administered in an amount necessary for administration. As a result, the administered substance A is administered into the cribriform foramen X3 from the distal end opening portion 14 of the puncture portion 12. After administering the administered substance A, the user removes the intranasal administration device 1 from the nasal cavity Z1 and completes the series of processing.

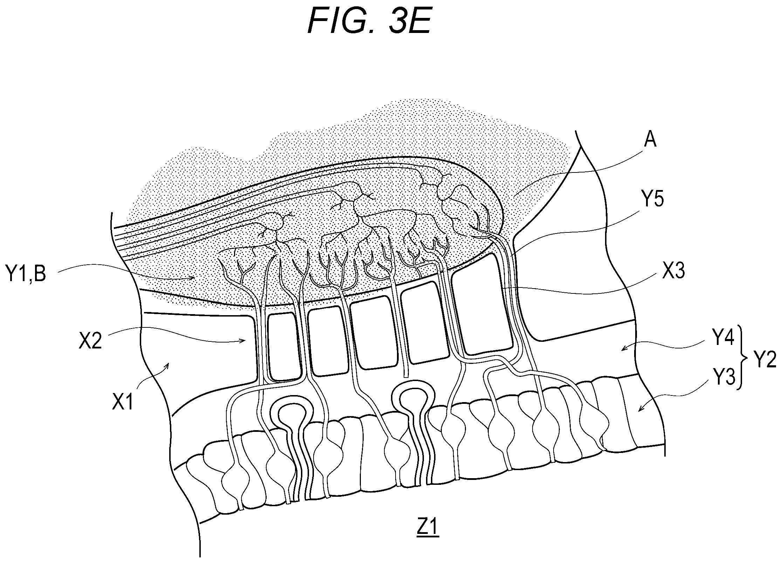

As illustrated in Fig. 3E, the administered substance A administered into the cribriform foramen X3 flows toward the olfactory-bulb-side opening of the cribriform foramen X3 and is delivered to the brain tissue B via a delivery medium such as the cerebrospinal fluid C or the olfactory nerve Y5. In addition, in Figs. 3D and 3E, illustration of the cerebrospinal fluid C is omitted in order to make the flow after administration of the administered substance A easier to understand.

As described above, the intranasal administration device 1 according to the present embodiment is an intranasal administration device 1 including the needle portion 10 having the puncture portion 12 for delivering the administered substance A intranasally into the brain of a mammal, in which the administered substance A is administered into the brain through an opening portion of the puncture portion 12 in a state where the puncture portion 12 is disposed within the cribriform foramen X3 and the ratio of the puncture length of the distal end of the opening portion (distal end opening portion 14) of the puncture portion 12 into the cribriform foramen X3 to the total length between the nasal-cavity-side opening and the olfactory-bulb-side opening of the cribriform foramen X3 is 60% or more and 100% or less.

With such a configuration, by administering the administered substance A such as a drug through the distal end opening portion 14 in a state where the distal end opening portion 14 of the puncture portion 12 is disposed within the cribriform foramen X3 and the puncture ratio of the distal end of the distal end opening portion 14 relative to the total length of the cribriform foramen X3 is controlled to 60% or more and 100% or less, the administered substance A can be delivered to the brain tissue B in a minimally invasive manner via a delivery medium such as the cerebrospinal fluid C or the olfactory nerve Y5. In particular, the present invention is extremely effective because a therapeutic macromolecular drug such as a protein or antibody, which is currently difficult to administer to brain tissue, can be efficiently delivered to the brain tissue while bypassing the blood-brain barrier or the like. In addition, since the administered substance A is administered within the cribriform foramen X3 formed in the cribriform plate X2 after passing through the olfactory mucosa Y2, where a large number of blood vessels and lymphatic vessels are present, the amount of the administered substance A flowing into blood vessels and lymphatic vessels can be minimized while suppressing leakage into the nasal cavity Z1. Furthermore, compared with the intrathecal administration, the intranasal spray administration, and the intraventricular administration, which have conventionally been known as methods for administering the administered substance A to the brain tissue B, the present invention can transfer the administered substance A to the brain in a minimally invasive manner and in an extremely efficient manner.

The intranasal administration system 200 according to the present embodiment includes the above-described intranasal administration device 1 and the guide catheter 100 having the lumen 111 into and from which at least a part of the intranasal administration device 1 can be inserted and removed, the guide catheter 100 guiding the needle portion 10 of the intranasal administration device 1 inserted into the lumen 111 to the cribriform foramen X3 of the cribriform plate X2.

With such a configuration, when the intranasal administration device 1 is inserted into the nasal cavity Z1, the puncture portion 12 of the needle portion 10 can be guided to the cribriform foramen X3, and therefore the intranasal administration operation can be performed safely and accurately.

### Examples

The effects of the present invention will be described using the following Examples and Comparative Examples. However, the technical scope of the present invention is not limited only to the following Examples.

### [Test 1]

In Test 1, using rat cadavers as subjects, intranasal administration was performed according to the puncture ratio of the puncture portion relative to the cribriform foramen of the cribriform plate, and the presence or absence of leakage of the administered substance into the nasal cavity was evaluated for each puncture ratio.

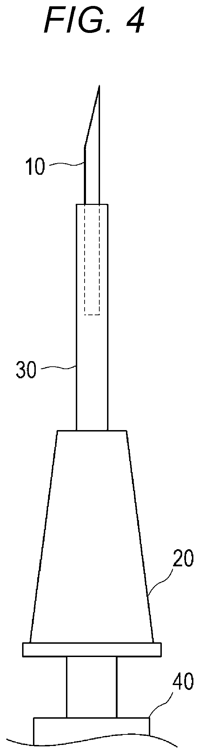

The intranasal administration device of Test 1 had the following specifications. Fig. 4 illustrates a configuration view of the intranasal administration device produced in Test 1. In Test 1, three types of devices having different exposed lengths of the needle tip (exposed lengths: 1.1 mm, 2 mm, and 2.5 mm) were prepared as intranasal administration devices.

For the needle portion 10, a 27G needle with a hub having an outer diameter of 0.4 mm and an inner diameter of 0.23 mm (product name: Blunt Needle 27G × 1 1/2, manufactured by Nipro Corporation) was cut at 15 mm from the proximal end, and a needle tip (having an outer diameter of 0.1 mm, an inner diameter of 0.06 mm, a total length of 15 mm, and a blade surface length of 0.1 mm, and produced from a material SUS304) was bonded to the 27G needle to have an exposed length of 1.1 mm (1100 µm). A microsyringe (product name: Gastight Syringe 1710TLL, manufactured by Hamilton Company) was attached to the proximal end side of the hub portion 20 as the accommodation portion 40. Similarly, devices in which the exposed length of the needle tip of the needle portion was 2 mm (2000 µm) and 2.5 mm (2500 µm), respectively, were produced. The exposed length of the needle tip corresponds to the total length of the puncture portion. The cannula portion 30 was the cut 27G needle.

Test 1 was performed according to the following test procedure.

First, male rats (slc: SD rats, 19 to 26 weeks of age, manufactured by Japan SLC, Inc.), which are subjects, were euthanized with carbon dioxide gas. The distal end part of the nose was resected using a router such that the puncture portion could reach a site deep in the olfactory epithelium where olfactory nerves converge.

Next, using a desktop precision universal testing machine (product name: Autograph AGS-X 1kNX, manufactured by Shimadzu Corporation) and a force transducer (product name: SSM-DAM-1000N, manufactured by Shimadzu Corporation), the needle portion of the device was pressed against the olfactory epithelium in one nasal cavity of the subject at 0.8 N and stopped at that position.

Next, using a compact desktop testing machine (product name: Autograph EZ-SX 500N, manufactured by Shimadzu Corporation) and a force transducer (model number: SMT1-5N, manufactured by Shimadzu Corporation), the above-described microsyringe was operated, 30 µL of a contrast agent (product name: Isovist Injection 240, Bayer Yakuhin, Ltd.) was administered at 20 µL/min, and the subject was left to stand for 5 minutes. The cribriform foramen was defined as a cribriform foramen (having a total length of approximately 2 mm) near the skull, where the olfactory nerves converge on the deep side of the olfactory epithelium.

After being left to stand for 5 minutes, each specimen was contrasted using a desktop micro-CT system (product name: skyscan1272, manufactured by Bruker Japan K.K.), and the presence or absence of leakage of the contrast agent into the nasal cavity was confirmed. The measurement conditions of the micro-CT system were a rotation of 0.5°, 360° imaging, a filter of Ai 0.5 mm + Cu 0.038 mm, the number of pixels of 1344 × 896, and a resolution of 15um, and the reconstruction conditions were a contrast threshold of 0 to 0.055 (Log). From the contrast image, the positions of the skull and the cribriform plate and the position of the needle portion were confirmed, and it was confirmed whether the distal end opening portion of the puncture portion was disposed at a location within the cribriform plate where olfactory nerves converge, that is, within the cribriform foramen.

In "Example" and "Comparative Example" of Test 1, it was considered that a contrast agent, which is an administered substance, was administered in a state where the needle portion was punctured toward a location where olfactory nerves converge without confirming the puncture state of the needle portion in real time, and the contrast agent was administered in a state where the puncture portion of the needle portion was punctured into the cribriform foramen based on confirmation of an image obtained by contrast imaging with a micro-CT system. In addition, regarding the difference in puncture ratio between the Example and the Comparative Example, although the puncture pressure was constant (0.8 N) in both the Example and the Comparative Example, the difference in the puncture ratio relative to the cribriform foramen is considered to have occurred due to a problem such as the puncture angle of the needle portion with respect to the cribriform plate.

The results of Test 1 were as follows.

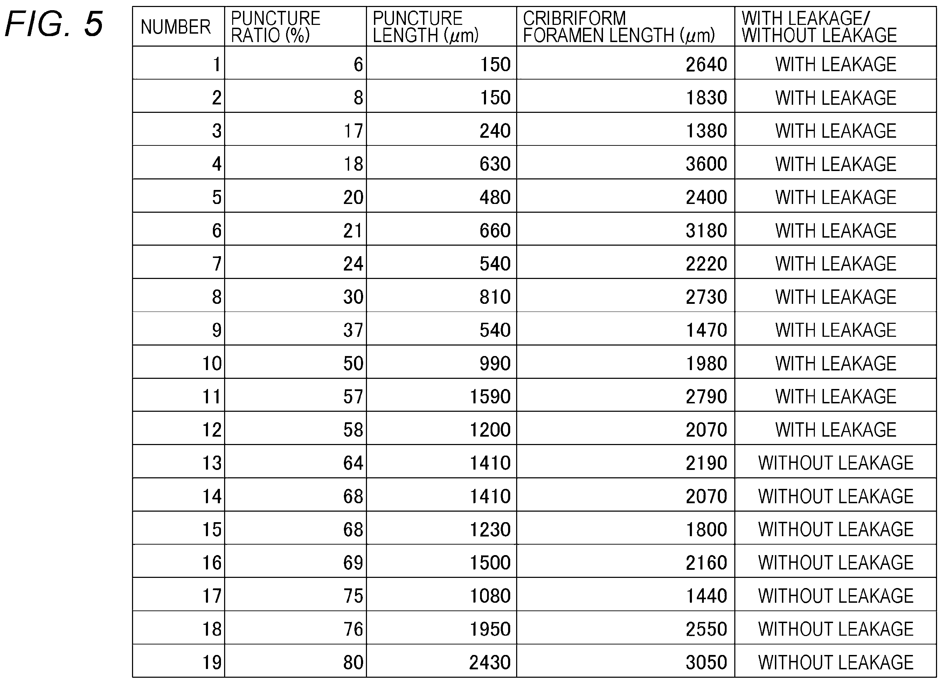

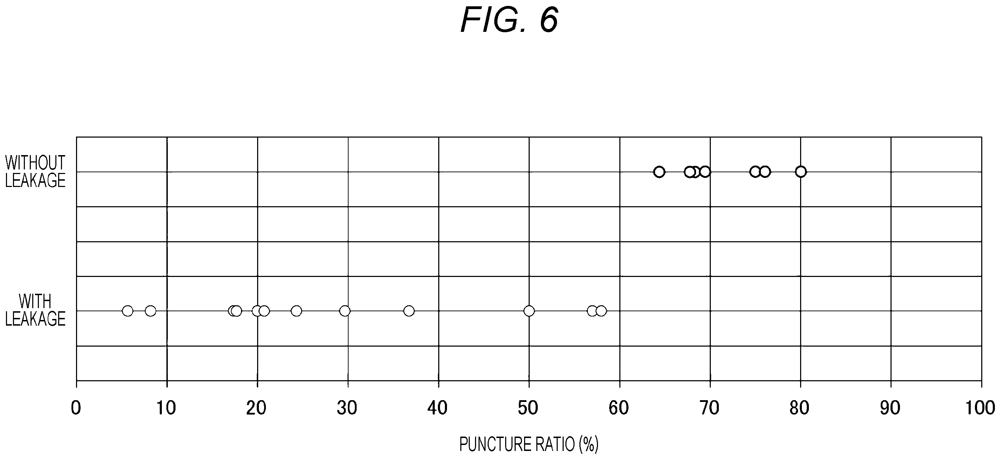

Fig. 5 is a table showing results of Test 1, and Fig. 6 is a graph illustrating results of Test 1. In the table of Fig. 5, the puncture length (µm) for each sample number was defined as the length from the distal end of the puncture portion punctured into the cribriform foramen, the cribriform foramen length (µm) was defined as the length of the shortest portion between the nasal-cavity-side opening and the olfactory-bulb-side opening of the cribriform foramen, and the puncture ratio (%) was defined as the ratio of the puncture length of the puncture portion to the cribriform foramen length (puncture length/cribriform foramen length). The graph illustrated in Fig. 6 plots the presence or absence of leakage into the nasal cavity. The point distribution in the lower part of the graph indicates samples with leakage into the nasal cavity, and the point distribution in the upper part of the graph indicates samples without leakage into the nasal cavity.

As illustrated in Figs. 5 and 6, Samples 13 to 19 had puncture ratios of 60% or more and 100% or less, and Samples 1 to 12 had puncture ratios of less than 60%. In addition, as illustrated in Fig. 6, because the distribution of the presence or absence of leakage into the nasal cavity was clearly separated between Samples 1 to 12 and Samples 13 to 19, Samples 13 to 19 were defined as Examples and Samples 1 to 12 were defined as Comparative Examples. Based on the results of Figs. 5 and 6, it was confirmed that "the distal end opening portion of the puncture portion is disposed in a cribriform foramen of the cribriform plate and the ratio of the puncture length of the distal end of the distal end opening portion into the cribriform foramen to the total length of the cribriform foramen is set to 60% or more and 100% or less" serves as one criterion for achieving 100% successful intranasal administration into the brain without leakage of the administered substance (contrast agent), which was administered, into the nasal cavity.

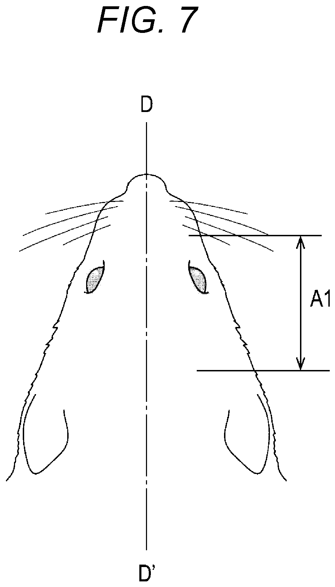

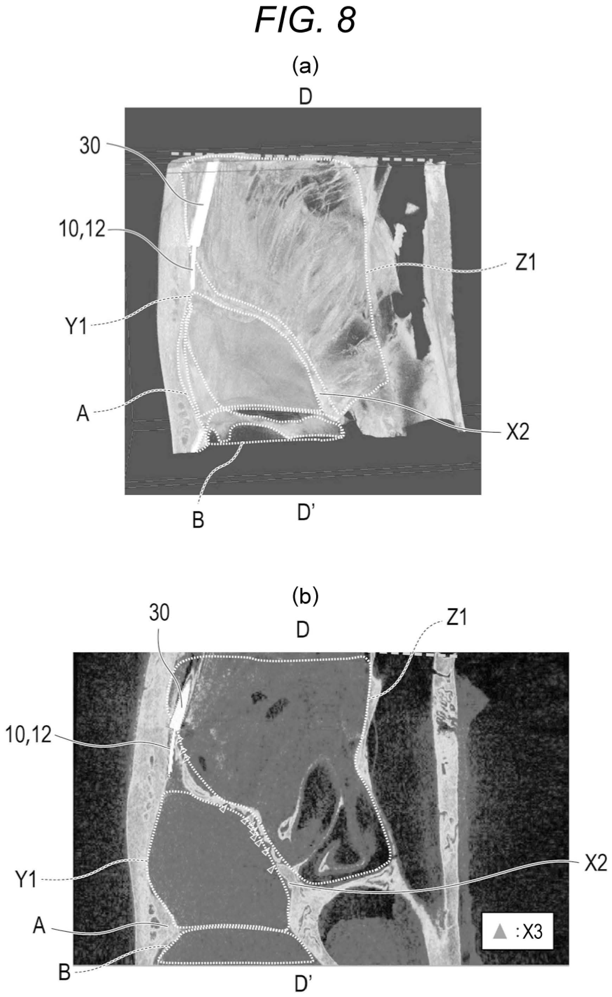

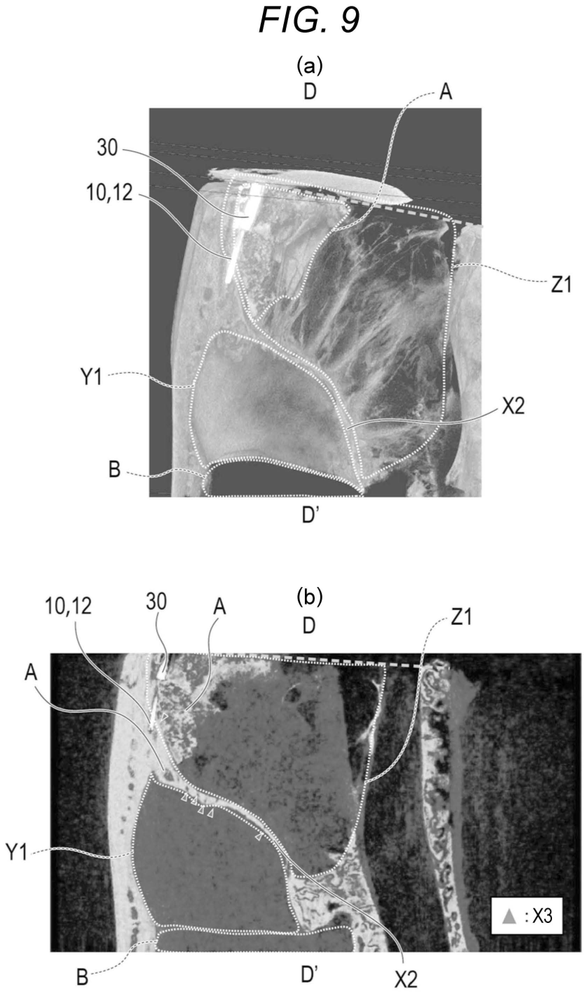

Fig. 7 is a view illustrating a cross-sectional position when imaging test results of Test 1, Fig. 8 is a photograph after administration of an administered substance (contrast agent) to Sample 14, which is an Example of Test 1, and Fig. 9 is a photograph after administration of an administered substance (contrast agent) to Sample 5, which is a Comparative Example of Test 1. As illustrated in Fig. 7, the photograph of the Example illustrated in Fig. 8 and the photograph of the Comparative Example illustrated in Fig. 9 are photographs taken when cut along the cutting line (line D-D') extending from the oral side toward the occipital region side of the rat, which is a subject. Fig. 8(a) is a CT image for explaining the distribution state of the contrast agent in the Example, and Fig. 8(b) is a CT image for explaining the puncture state of the intranasal administration device in the Example. Fig. 9(a) is a CT image for explaining the distribution state of the contrast agent in the Comparative Example, and Fig. 9(b) is a CT image for explaining the puncture state of the intranasal administration device in the Comparative Example. Note that the images illustrated in Figs. 8(a) and 9(a) are 3D data, the images illustrated in Figs. 8(b) and 9(b) are 2D data, and all are images in the vicinity of Position A1 illustrated in Fig. 7.

Since Sample 14, which is the Example, was administered in a state where the distal end opening portion of the puncture portion 12 was punctured into the cribriform foramen X3 of the cribriform plate X2 at a puncture ratio of 68% as illustrated in Fig. 8(b), the contrast agent, which is the administered substance A, was transferred to the brain tissue B side as illustrated in Fig. 8(a), and no leakage into the nasal cavity Z1 was confirmed. In contrast, since Sample 5, which is the Comparative Example, was administered in a state where the distal end opening portion of the puncture portion 12 was punctured at a puncture ratio of 20% as illustrated in Fig. 9(b), most of the contrast agent after administration leaked into the nasal cavity Z1 as illustrated in Fig. 9(a), and transfer to the brain tissue B side was hardly confirmed.

As described above, from the results of Test 1, it was confirmed that the administered substance A administered in a state where the distal end opening portion 14 of the puncture portion 12 was disposed in the cribriform foramen X3 formed in the cribriform plate X2 and the puncture ratio of the distal end of the distal end opening portion 14 relative to the total length of the cribriform foramen X3 was 60% or more and 100% or less was administered to the brain tissue B side without leaking into the nasal cavity Z1. Because the cribriform foramen X3 has a nasal-cavity-side opening and an olfactory-bulb-side opening and communicates only with the nasal cavity Z1 side and the olfactory bulb Y1 side, when the administered substance A does not leak into the nasal cavity Z1, it can be determined that the administered substance A has been delivered to the olfactory bulb Y1 side, which is the brain tissue B. Accordingly, it can be said that, when the administered substance A is administered using the intranasal administration device 1 of the present invention in a state where the distal end opening portion 14 of the puncture portion 12 is disposed in the cribriform foramen X3 of the cribriform plate X2 and the puncture ratio of the distal end of the distal end opening portion 14 relative to the total length of the cribriform foramen X3 is 60% or more and 100% or less, the administered substance A can be administered with a liquid pressure opposing the flow of the cerebrospinal fluid C seeping from the cribriform foramen X3, and can be efficiently delivered to the brain tissue B while preventing leakage into the nasal cavity Z1. Further, since the structures of the cribriform foramina X3 of rats, which are the subjects in Test 1, and humans are equivalent to each other, the results of Test 1 show that, when the administered substance A is administered intranasally to the brain of a human using the intranasal administration device of the present invention in a state where the puncture ratio of the needle portion 10 relative to the cribriform foramen X3 is 60% or more and 100% or less, the administered substance A can be efficiently delivered to the brain tissue B.

### [Test 2]

In Test 2, using living cynomolgus monkeys as subjects, intranasal administration was performed according to the puncture ratio of the puncture portion relative to the cribriform foramen of the cribriform plate, and the presence or absence of leakage of the administered substance into the nasal cavity was evaluated for each puncture ratio.

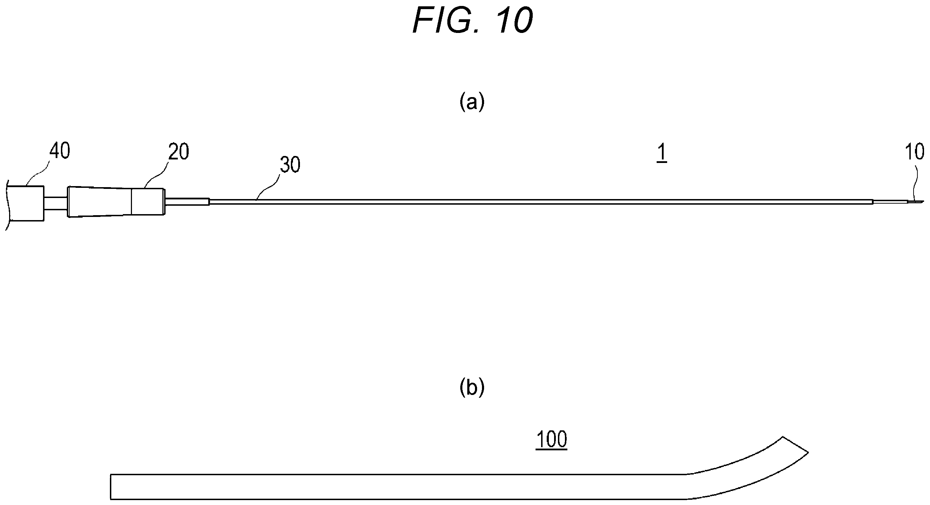

The intranasal administration device of Test 2 had the following specifications. Fig. 10 shows configuration views of the intranasal administration device 1 (illustrated in Fig. 10(a)) and the guide catheter 100 (illustrated in Fig. 10(b)) produced in Test 2.

As the intranasal administration device 1, five types of devices having different exposed lengths of the needle tip (exposed lengths: 0.3 mm, 1 mm, 2 mm, 2.4 mm, and 3 mm) were prepared.

First, an inner tube catheter having an outer diameter of 0.47 mm, an inner diameter of 0.37 mm, and a total length of 165 mm (produced using PEEK as the material) was inserted into an outer tube catheter having an outer diameter of 0.6 mm, an inner diameter of 0.52 mm, and a total length of 155 mm (produced using PEEK as the material), and the inner tube catheter was bonded with a UV adhesive in a state of being exposed by 1 mm toward the distal end side, thereby producing a double-tube catheter. Next, an 18G needle with a hub (product name: Terumo Non-Bevel Needle 18G 1 1/2, manufactured by Terumo Corporation) was cut at a position 10 mm from the proximal end, and the proximal end side of the produced double-tube catheter was fitted to the distal end side of the hub and bonded with a UV adhesive. Next, the inner tube catheter was cut such that the inner tube catheter was exposed by 7 mm from the outer tube catheter, and the step between the catheters was filled and smoothed with an epoxy adhesive (product name: Bond Quick 30, manufactured by Konishi Co., Ltd.). The hub portion 20 of the produced intranasal administration device is the hub part of the above-described 18G needle with a hub, and the cannula portion 30 is constituted by the double-tube catheter.

A needle tip (having an outer diameter of 0.1 mm, an inner diameter of 0.06 mm, a total length of 15 mm, and a blade surface length of 0.1 mm, and produced from a material SUS304) was disposed and bonded with a UV adhesive such that the exposed lengths from the distal end of the inner tube catheter in the cannula portion 30 having the above configuration were 0.3 mm, 1 mm, 2 mm, 2.4 mm, and 3 mm, thereby producing five types of devices. A microsyringe (product name: Gastight Syringe 1710TLL, manufactured by Hamilton Company) was attached to the proximal end side of the hub portion 20 of each device as the accommodation portion 40.

The produced devices were inserted into the nasal cavity using the guide catheter 100 as an insertion aid. As the guide catheter 100, a guide catheter formed of SUS304 and processed to have a total length of 90 mm, an outer diameter of φ0.82, an inner diameter of φ0.68, and a bending angle of a curved portion on the distal end side of 45 degrees, or a bendable guide catheter in which a straight portion was provided with a spiral cut to impart flexibility, was used.

Test 2 was performed according to the following test procedure.

First, cynomolgus monkeys, which are subjects (3 years and 7 months to 3 years and 11 months old, male), were anesthetized. The anesthetic agents and administration methods were as follows.
- Induction anesthesia: Ketalar for intramuscular injection 500 mg, Administration route: intramuscular administration, Administration amount: 10 mg/kg (0.2 mL/kg).
- Maintenance anesthesia: Propofol injection 1% for animal "Mylan", Administration route: intravenous administration, Administration amount: 0.5 to 30 mg/kg/hr (0.05 to 3 mL/kg/hour).

Next, contrast imaging was performed using a 16-channel X-ray CT apparatus (product name: Bright Speed Elite, manufactured by GE Healthcare), and the guide catheter was inserted into one nasal cavity of the subject while confirming contrast images. At the time of insertion, the distal end opening of the guide catheter was directed toward the cribriform plate side and the guide catheter was adjusted such that the distal end was positioned immediately below the cribriform plate.

Next, the produced device was inserted into the guide catheter, the olfactory mucosa was punctured by an amount corresponding to the exposed length of the puncture portion, and the above-described microsyringe was operated to administer 30 µL of a contrast agent (product name: Isovist Injection 240, Bayer Yakuhin, Ltd.) at 20 µL/min using a microsyringe pump (product name: IC3200, manufactured by KD Scientific). Immediately after administration, the contrast agent was imaged using the above-described X-ray CT apparatus, and it was confirmed that the contrast agent had been administered to the olfactory mucosa. In addition, by confirming that the guide catheter had not moved, it was confirmed that the puncture portion was in a state of being punctured into the cribriform foramen.

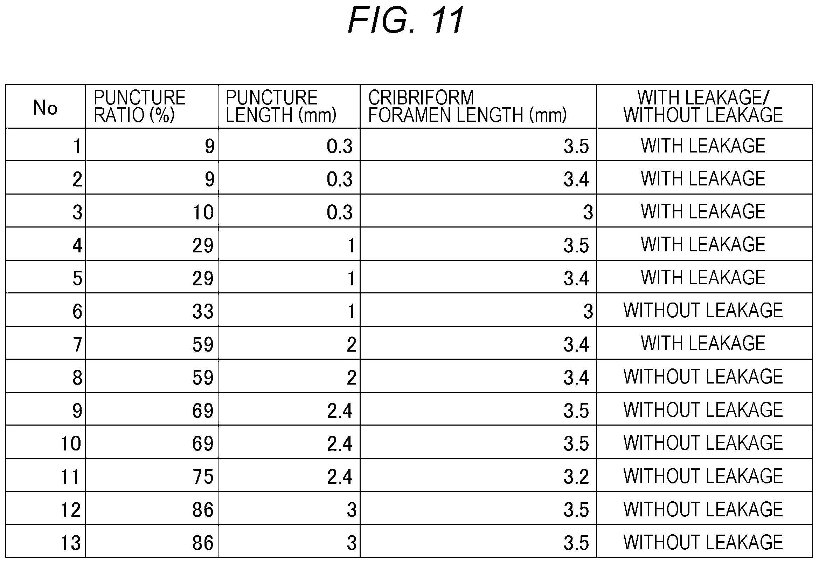

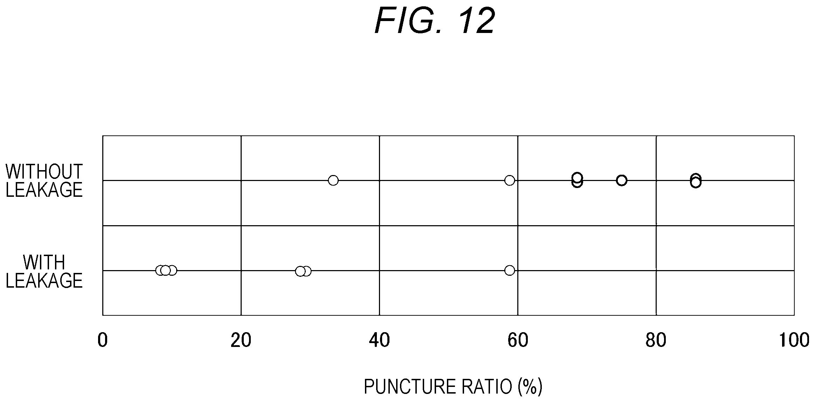

The test results are shown in Figs. 11 and 12.

In the table of Fig. 11, the puncture length (µm) for each sample number was defined as the length from the distal end of the puncture portion punctured into the cribriform foramen, the cribriform foramen length (µm) was defined as the length of the shortest portion between the nasal-cavity-side opening and the olfactory-bulb-side opening of the cribriform foramen, and the puncture ratio (%) was defined as the ratio of the puncture length of the puncture portion to the cribriform foramen length (puncture length/cribriform foramen length). The graph illustrated in Fig. 12 plots the presence or absence of leakage into the nasal cavity. The point distribution in the lower part of the graph indicates samples with leakage into the nasal cavity, and the point distribution in the upper part of the graph indicates samples without leakage into the nasal cavity.

As illustrated in Figs. 11 and 12, Samples 9 to 13 had puncture ratios of 60% or more and 100% or less, and Samples 1 to 8 had puncture ratios of less than 60%. In addition, as illustrated in Fig. 11, because the distribution of the presence or absence of leakage into the nasal cavity was clearly separated between Samples 1 to 8 and Samples 9 to 13, Samples 9 to 13 were defined as Examples and Samples 1 to 8 were defined as Comparative Examples. Based on the results of Figs. 11 and 12, it was confirmed that "the distal end opening portion of the needle portion is disposed in a cribriform foramen of the cribriform plate and the ratio of the puncture length of the distal end of the distal end opening portion into the cribriform foramen to the total length of the cribriform foramen is set to 60% or more and 100% or less" serves as one criterion for achieving 100% successful intranasal administration into the brain without leakage of the administered substance (contrast agent), which was administered, into the nasal cavity.

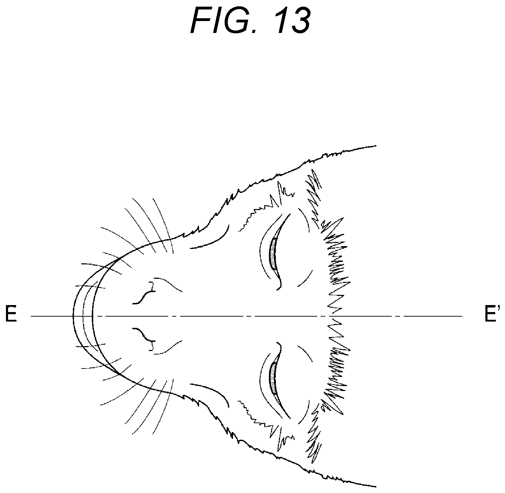

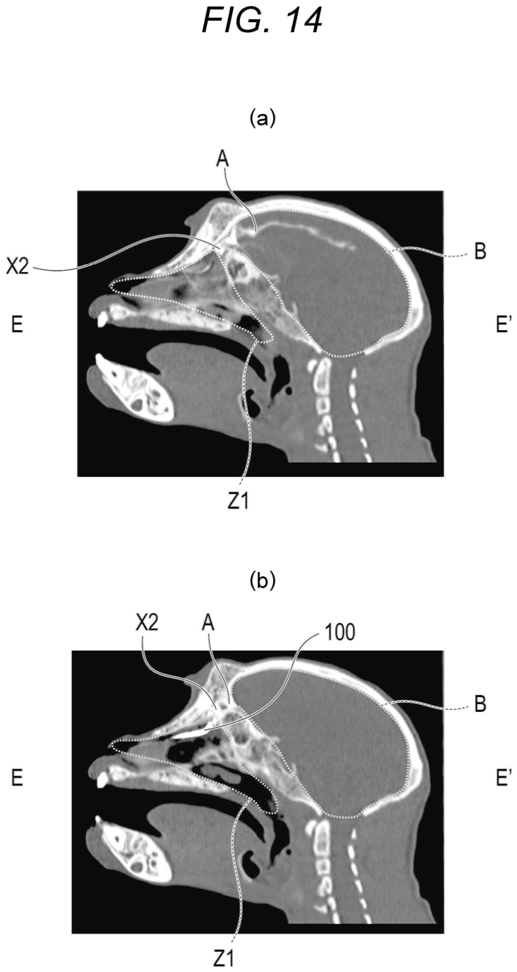

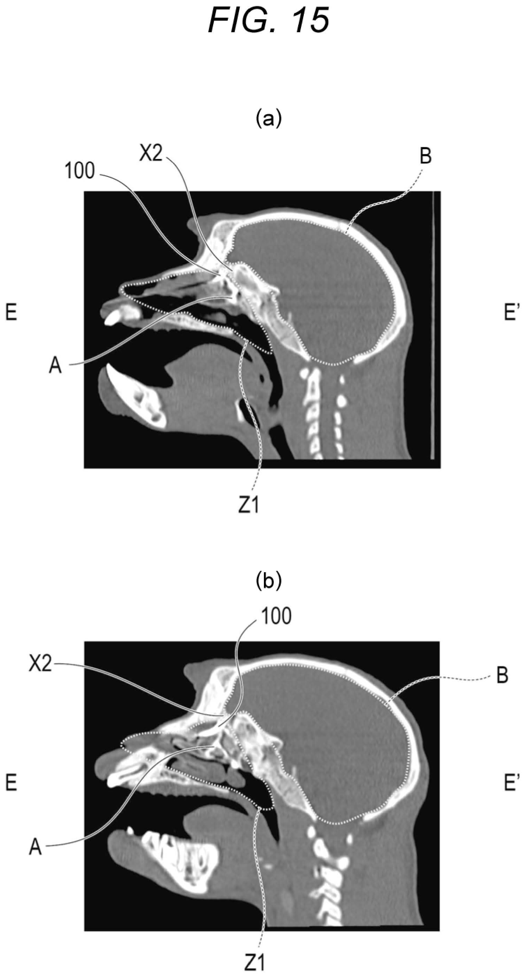

Fig. 13 is a view illustrating a cross-sectional position when imaging test results of Test 2, Fig. 14 is a photograph after administration of an administered substance (contrast agent) to Sample 12, which is an Example of Test 1, and Fig. 15 is a photograph after administration of an administered substance (contrast agent) to Sample 3, which is a Comparative Example of Test 1. As illustrated in Fig. 13, the photograph of the Example illustrated in Fig. 14 and the photograph of the Comparative Example illustrated in Fig. 15 are photographs taken when cut along the cutting line (line E-E') extending from the oral side toward the occipital region side of a cynomolgus monkey, which is a subject. Fig. 14(a) is a CT image for explaining the distribution state of the contrast agent in the Example, and Fig. 14(b) is a CT image for explaining the puncture state of the intranasal administration device in the Example. Fig. 15(a) is a CT image for explaining the distribution state of the contrast agent in the Comparative Example, and Fig. 15(b) is a CT image for explaining the puncture state of the intranasal administration device in the Comparative Example.

Since Sample 12, which is the Example, was administered at a puncture ratio of the puncture portion of 86% as illustrated in Fig. 14(b), the contrast agent was transferred to the brain tissue B side as illustrated in Fig. 14(a), and no leakage into the nasal cavity was confirmed. In contrast, since Sample 3, which is the Comparative Example, was administered at a puncture ratio of the puncture portion of 10% as illustrated in Fig. 15(b), the contrast agent after administration leaked into the nasal cavity as illustrated in Fig. 15(a), and transfer to the brain tissue B side could not be confirmed.

In Test 2, Sample 6 (puncture ratio: 33%) resulted in no leakage. It is considered that the cynomolgus monkeys, which are the subjects in Test 2, had cribriform foramina about 2.5 times larger in volume than those of the rats, which are the subjects in Test 1, and because the properties of the olfactory nerves differ among individuals and are susceptible to individual differences, no leakage into the nasal cavity occurred even when the puncture ratio was less than 60%.

As described above, from the results of Test 2, it was confirmed that the administered substance A administered in a state where the distal end opening portion 14 of the puncture portion 12 was disposed in the cribriform foramen X3 formed in the cribriform plate X2 and the puncture ratio of the distal end of the distal end opening portion 14 relative to the total length of the cribriform foramen X3 was 60% or more and 100% or less was administered to the brain tissue B side without leaking into the nasal cavity Z1. Because the cribriform foramen X3 has a nasal-cavity-side opening and an olfactory-bulb-side opening and communicates only with the nasal cavity Z1 side and the olfactory bulb Y1 side, when the administered substance A does not leak into the nasal cavity Z1, it can be determined that the administered substance A has been delivered to the olfactory bulb Y1 side, which is the brain tissue B. Accordingly, it can be said that, when the administered substance A is administered using the intranasal administration device 1 of the present invention in a state where the distal end opening portion 14 of the puncture portion 12 is disposed in the cribriform foramen X3 of the cribriform plate X2 and the puncture ratio of the distal end of the distal end opening portion 14 relative to the total length of the cribriform foramen X3 is 60% or more and 100% or less, the administered substance A can be administered with a liquid pressure opposing the flow of the cerebrospinal fluid C seeping from the cribriform foramen X3, and can be efficiently delivered to the brain tissue B while preventing leakage into the nasal cavity Z1. Further, since the structures of the cribriform foramina X3 of the cynomolgus monkeys, which are the subjects in Test 2, and humans are equivalent to each other, the results of Test 2 show that, when the administered substance A is administered intranasally to the brain of a human using the intranasal administration device of the present invention in a state where the puncture ratio of the needle portion 10 relative to the cribriform foramen X3 is 60% or more and 100% or less, the administered substance A can be efficiently delivered to the brain tissue B.

In addition, Sample 7, which was defined as a Comparative Example, had a puncture ratio of 59% and resulted in leakage into the nasal cavity, whereas Sample 8 had the same puncture ratio of 59% as Sample 7 and resulted in no leakage into the nasal cavity. From the results of Test 2, it was confirmed that, when the puncture ratio exceeds 60%, transfer to the brain tissue B side can be achieved with a 100% success rate without leakage into the nasal cavity, but it was also confirmed that, as in Samples 7 and 8, even when the puncture ratio of the distal end opening portion is less than 60%, administration can be performed without leakage into the nasal cavity at a high probability of 50%. Therefore, administration of an administered substance using the intranasal administration device of the present invention is considered to be less invasive than currently known administration methods such as the intranasal spray method, the intraventricular administration method, and the intrathecal administration method, to have an excellent transfer rate to the brain tissue B, and to be capable of rapidly delivering a sufficient administration amount to the brain, even when the puncture ratio of the distal end opening portion relative to the total length of the cribriform foramen is less than 60% (for example, 50% or more and 100% or less). However, when administration to humans is assumed, conditions that achieve a 100% success rate are important, and therefore it can be said that the puncture ratio is optimally 60% or more and 100% or less.

### [Test 3]

In Test 3, using living cynomolgus monkeys as subjects, intranasal administration was performed according to the puncture ratio of the puncture portion 12 relative to the cribriform foramen X3 of the cribriform plate X2, and the presence or absence of leakage of the administered substance A into the nasal cavity Z1 was evaluated for each puncture ratio.

A difference from Test 2 was that a needle tip (having an outer diameter of 0.1 mm, an inner diameter of 0.09 mm, a total length of 15 mm, and a blade surface length of 0.1 mm, and produced from a material SUS304) was disposed and bonded with a UV adhesive such that the exposed length from the distal end of the inner tube catheter in the cannula portion 30 having the above configuration was 2.0 mm, thereby producing an intranasal administration device. A microsyringe (product name: Gastight Syringe 1001LT, manufactured by Hamilton Company) was attached to the proximal end side of the hub portion 20 of the device as the accommodation portion 40.

Next, the produced device was inserted into the guide catheter, the olfactory mucosa was punctured by an amount corresponding to the exposed length of the puncture portion, and the above-described microsyringe was operated to administer 0.5 mL of a contrast agent (product name: Gadovist Intravenous Injection, Bayer Yakuhin, Ltd.) at 20 µL/min using a microsyringe pump (product name: IC3200, manufactured by KD Scientific). In addition, when carrying out Test 3, treatment of the subject and placement of the guide catheter were the same as in Test 2.

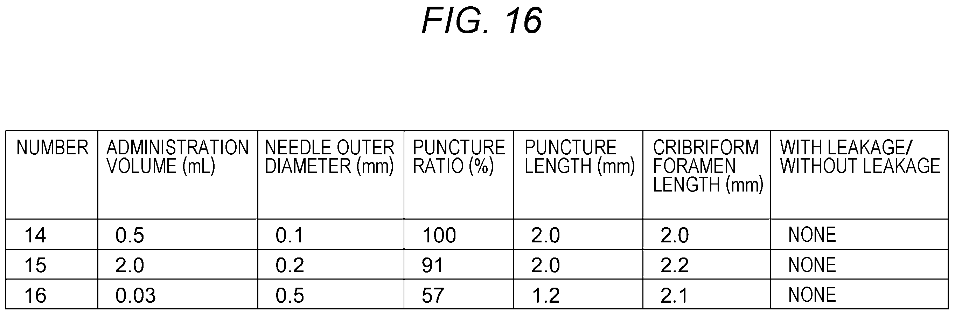

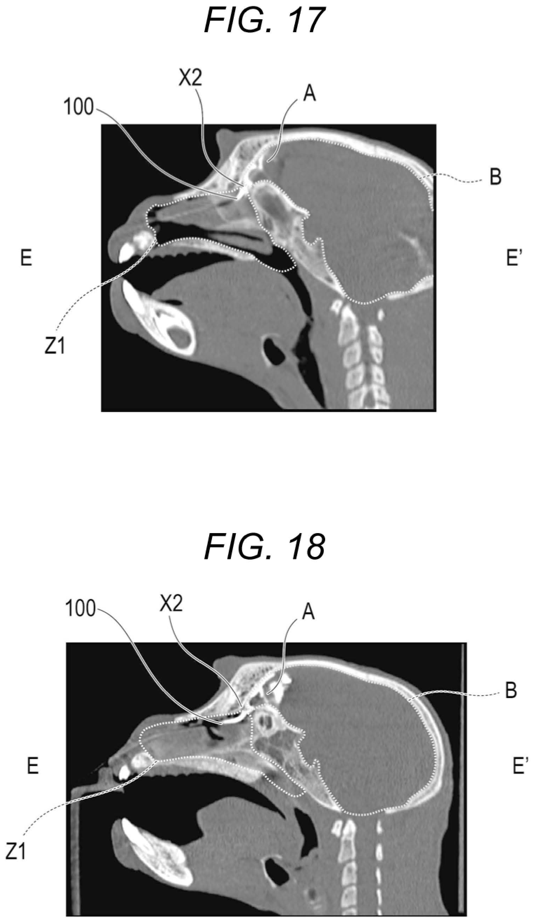

Fig. 16 shows the test results of Test 3. Fig. 17 is a CT image for explaining the distribution state of the contrast agent when cut along the cutting line (line E-E') extending from the oral side toward the occipital region side of the subject cynomolgus monkey illustrated in Fig. 13. Since Sample 14, which is the Example, was administered at a puncture ratio of the puncture portion relative to the cribriform foramen X3 (cribriform foramen length: 2.0 mm) of 100% as illustrated in Fig. 16, the contrast agent, which is the administered substance A, was transferred to the brain tissue B side as illustrated in Fig. 17, and no leakage into the nasal cavity was confirmed.

### [Test 4]

In Test 4, using living cynomolgus monkeys as subjects, intranasal administration was performed according to the puncture ratio of the puncture portion 12 relative to the cribriform foramen X3 of the cribriform plate X2, and the presence or absence of leakage of the administered substance A into the nasal cavity Z1 was evaluated for each puncture ratio.

A difference from Tests 2 and 3 was that a needle tip (having an outer diameter of 0.2 mm, an inner diameter of 0.09 mm, a total length of 15 mm, and a blade surface length of 0.1 mm, and produced from a material SUS304) was disposed and bonded with a UV adhesive such that the exposed length from the distal end of the inner tube catheter in the cannula portion 30 having the above configuration was 2.0 mm, thereby producing an intranasal administration device. A microsyringe (product name: Gastight Syringe 1002TLL, manufactured by Hamilton Company) was attached to the proximal end side of the hub portion 20 of the device as the accommodation portion 40.

Next, the produced device was inserted into the guide catheter, the olfactory mucosa was punctured by an amount corresponding to the exposed length of the puncture portion, and the above-described microsyringe was operated to administer 2.0 mL of a contrast agent (product name: Omnipaque 240 Injection, GE Healthcare Pharma Corporation) at 20 µL/min using a microsyringe pump (product name: IC3200, manufactured by KD Scientific). In addition, when carrying out Test 4, treatment of the subject and placement of the guide catheter were the same as in Test 2.

Fig. 16 shows the test results of Test 4. Fig. 18 is a CT image for explaining the distribution state of the contrast agent when cut along the cutting line (line E-E') extending from the oral side toward the occipital region side of the subject cynomolgus monkey illustrated in Fig. 13. Since Sample 15, which is the Example, was administered at a puncture ratio of the puncture portion relative to the cribriform foramen X3 (cribriform foramen length: 2.2 mm) of 91% as illustrated in Fig. 16, the contrast agent, which is the administered substance A, was transferred to the brain tissue B side as illustrated in Fig. 18, and no leakage into the nasal cavity was confirmed.

### [Test 5]

In Test 5, using a living cynomolgus monkey (5 years and 5 months old, male) as a subject, intranasal administration was performed according to the puncture ratio of the puncture portion 12 relative to the cribriform foramen X3 of the cribriform plate X2, and the presence or absence of leakage of the administered substance A into the nasal cavity Z1 was evaluated for each puncture ratio.

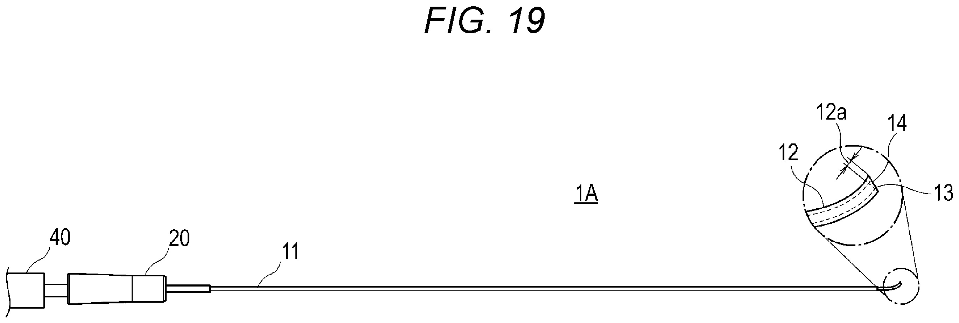

As illustrated in Fig. 19, an intranasal administration device 1A used in Test 5 is configured to include the needle portion 10, the hub portion 20, and the accommodation portion 40. The intranasal administration device 1A differs from the device configuration illustrated in Fig. 10a in that the cannula portion 30 is omitted, and, in order to directly puncture the cribriform foramen X3 of the cribriform plate X2 without using the guide catheter 100, the distal end side of the puncture portion 12 is curved in advance. The intranasal administration device 1A was produced as follows.

First, a SUS pipe having an outer diameter of 0.2 mm, an inner diameter of 0.09 mm, and a total length of 200 mm was inserted into a SUS pipe having an outer diameter of 0.5 mm, an inner diameter of 0.36 mm, and a total length of 100 mm, and the distal ends thereof were aligned on the same surface and bonded with an epoxy adhesive (product name: Bond Quick 30, manufactured by Konishi Co., Ltd.), thereby forming a double-tube SUS pipe. Next, a 22G needle with a hub (product name: Terumo Non-Bevel Needle 22G 1 1/2, manufactured by Terumo Corporation) was cut at a position 10 mm from the proximal end, and the proximal end side of the produced double-tube SUS pipe was fitted to the distal end side of the hub and bonded with a UV adhesive. The hub portion 20 of the device is the hub part of the above-described 22G needle with a hub, and the needle shaft portion 11 is constituted by the double-tube SUS pipe.

For the puncture portion 12, a needle tip (having an outer diameter of 0.5 mm, an inner diameter of 0.36 mm, a total length of 100 mm, and a blade surface length of 0.09 mm, and produced from a material SUS304) was disposed and bonded with a UV adhesive such that an exposed length thereof from the distal end of the needle shaft portion 11 having the above configuration was 2.0 mm, thereby producing the device. A microsyringe (product name: Gastight Syringe 1710TLL, manufactured by Hamilton Company) was attached to the proximal end side of the hub portion 20 of the device as the accommodation portion 40.

Next, the produced device was inserted into the nasal cavity of the monkey, and using the principle of leverage, the olfactory mucosa was punctured by an amount corresponding to the curved length of the puncture portion, and the above-described microsyringe was operated to administer 0.03 mL of a contrast agent (product name: Omnipaque 240 Injection, GE Healthcare Pharma Corporation) at 20 µL/min using a microsyringe pump (product name: IC3200, manufactured by KD Scientific). In addition, when carrying out Test 5, treatment of the subject was the same as in Test 2.

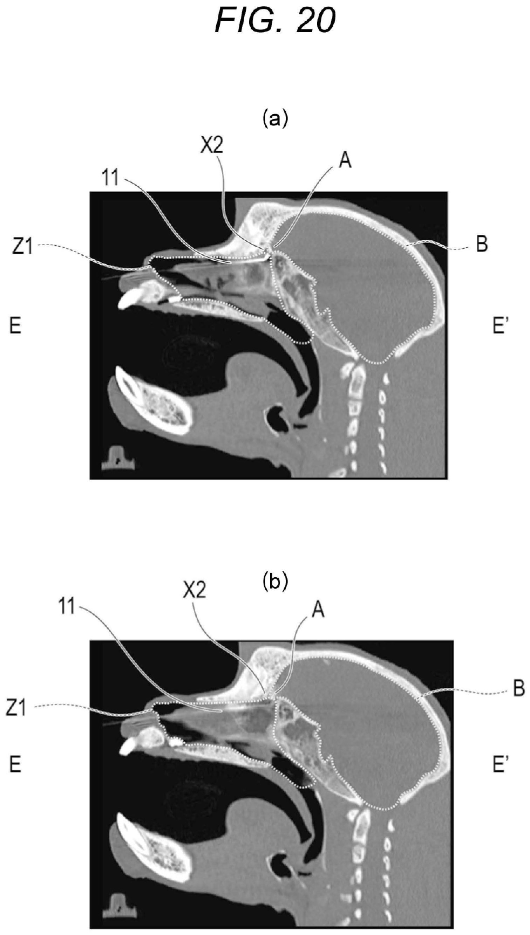

Fig. 16 shows the test results of Test 5. Fig. 20 is a CT image taken when cut along the cutting line (line E-E') extending from the oral side toward the occipital region side of the subject cynomolgus monkey illustrated in Fig. 13, in which Fig. 20(a) is a CT image for explaining the distribution state of the contrast agent in Test 5, and Fig. 20(b) is a CT image for explaining the puncture state of the intranasal administration device in Test 5. Sample 16, which is the Example, was administered at a puncture ratio of the puncture portion relative to the cribriform foramen X3 (cribriform foramen length: 2.1 mm) of 57% as illustrated in Fig. 16, but the contrast agent, which is the administered substance A, was transferred to the brain tissue B side as illustrated in Fig. 20(b), and no leakage into the nasal cavity was confirmed.

As described above, Samples 1 to 13 of the above-described Test 2 shown in Fig. 11 were Examples in which the outer diameter of the needle was 0.2 mm. However, as illustrated in Fig. 16, in Sample 16, when the outer diameter of the needle was 0.5 mm, no leakage into the nasal cavity was confirmed even at a puncture depth of less than 60%. In addition, as illustrated in Fig. 16, Samples 14 and 15 had deeper puncture ratios (91% and 100%) than those of the respective samples of Test 2 shown in Fig. 11, and therefore no leakage into the nasal cavity was confirmed.

The present application is based on Japanese Patent Application No. 2024-017856 filed on February 8, 2024, the disclosure of which is incorporated herein by reference in its entirety.

### Reference Signs List

- 1: Intranasal administration device
- 10: Needle portion (lumen 10a)
- 11: Needle shaft portion
- 12: Puncture portion (needle tip portion 12a)
- 13: Blade surface
- 14: Distal end opening portion
- 15: Proximal end opening portion
- 20: Hub portion
- 21: Main body portion (lumen 21a)
- 22: Connection portion
- 30: Cannula portion
- 31: Main body portion
- 32: Stopper portion (abutment portion 32a)
- 40: Accommodation portion
- 41: Accommodation space
- 42: Liquid feeding portion (distal end opening portion 42a)
- 50: Connection member
- 100: Guide catheter
- 110: Catheter main body
- 111: Lumen
- 200: Intranasal administration system
- A: Administered substance
- B: Brain tissue
- C: Cerebrospinal fluid (CSF)
- X1: Ethmoid bone
- X2: Cribriform plate
- X3: Cribriform foramen
- Y1: Olfactory bulb
- Y2: Olfactory mucosa
- Y3: Olfactory epithelium
- Y4: Lamina propria
- Y5: Olfactory nerve
- Z1: Nasal cavity

## Claims

1. An intranasal administration device comprising a needle portion having a puncture portion for delivering an administered substance intranasally into the brain of a mammal, wherein
the administered substance is administered into the brain through an opening portion of the puncture portion in a state where the opening portion is disposed within a cribriform foramen and a ratio of a puncture length of a distal end of the opening portion into the cribriform foramen to a total length between a nasal-cavity-side opening and an olfactory-bulb-side opening of the cribriform foramen is 60% or more and 100% or less.

2. The intranasal administration device according to claim 1, wherein
the intranasal administration device has a cannula portion that is constituted by a tubular member and is disposed to cover the needle portion such that the puncture portion is exposed,
the opening portion is provided on a distal end side of the puncture portion,
a stopper portion having an abutment portion that abuts against an olfactory epithelium of an olfactory mucosa is formed at a distal end portion of the cannula portion, and
the opening portion is disposed within the cribriform plate in a state where the abutment portion of the stopper portion is brought into contact with the olfactory epithelium.

3. The intranasal administration device according to claim 2, wherein
the intranasal administration device has a hub portion to which an accommodation portion accommodating the administered substance is attachable, and
the hub portion holds a proximal end of the cannula portion and/or a proximal end of a needle shaft portion of the needle portion inserted through a lumen of the cannula portion.

4. The intranasal administration device according to claim 1, wherein a needle tip portion of the puncture portion is an open end provided with a blade surface on a distal end side thereof, and a total length of the puncture portion is 0.35 mm or more and 5.4 mm or less.

5. The intranasal administration device according to claim 2, wherein a total length of the cannula portion is 55 mm or more and 410 mm or less.

6. The intranasal administration device according to claim 3, wherein
the abutment portion of the stopper portion is a distal end surface of the cannula portion that comes into contact with the olfactory epithelium,
a length of a longest portion in a radial direction of the abutment portion is 0.2 mm or more and 15 mm or less, and
an outer diameter on a proximal end side of the puncture portion is 0.1 mm or more and 2.1 mm or less.

7. The intranasal administration device according to claim 2, wherein a cross-sectional shape of the abutment portion of the stopper portion is circular or elliptical.

8. The intranasal administration device according to claim 1 or 2, wherein the intranasal administration device has an accommodation portion accommodating the administered substance and is configured such that the administered substance accommodated in the accommodation portion is dischargeable through the opening portion of the puncture portion.

9. An intranasal administration system comprising:
the intranasal administration device according to any one of claims 1 to 8; and
a guide catheter having a lumen into and from which at least a part of the intranasal administration device is insertable and removable, the guide catheter guiding the needle portion of the intranasal administration device inserted into the lumen to the cribriform foramen formed in the cribriform plate.
